# EUROPÄISCHE PATENTSCHRIFT

(11) **EP 3 880 641 B1**
(45) Veröffentlichungstag und Bekanntmachung des Hinweises auf die Patenterteilung: **15.03.2023**
(21) Anmeldenummer: 19702360.9
(22) Anmeldetag: 23.01.2019
(51) Int. Cl.: C07C 67/03, C07C 67/08, C07C 69/675, C11C 3/02, C12P 7/62, A23L 33/00, A61K 31/19, A61P 3/00

(54) **VERFAHREN ZUR HERSTELLUNG VON GLYCERIDEN VON HYDROXYCARBONSÄUREN**
METHOD FOR PRODUCING GLYCERIDES OF HYDROXY CARBOXYLIC ACIDS
PROCÉDÉ DE PRÉPARATION DE GLYCÉRIDES D'ACIDES HYDROCARBOXYLIQUES

(30) Priorität: 17.01.2019 WO PCT/EP2019/051116
(43) Veröffentlichungstag der Anmeldung: 22.09.2021
(73) Patentinhaber: KetoLipix Therapeutics GmbH, 20459 Hamburg (DE)
(72) Erfinder: LOCHMANN, Dirk, 58453 Witten (DE); REYER, Sebastian, 58453 Witten (DE); STEHR, Michael, 58453 Witten (DE)
(74) Vertreter: Strehlke, Ingo Kurt
(86) Internationale Anmeldenummer: PCT/EP2019/051539
(87) Internationale Veröffentlichungsnummer: WO 2020/147978

(56) Entgegenhaltungen:
- EP-A1- 0 736 256
- WO-A1-95/09144
- WO-A1-2008/005818
- WO-A1-2013/150153
- WO-A1-2018/118369
- WO-A1-2018/132189
- WO-A1-2018/195421
- WO-A1-2019/002828
- WO-A2-02/06368
- WO-A2-2006/034361
- JP-A- H0 383 950

## Beschreibung

Die vorliegende Erfindung betrifft das Gebiet der Ketokörper und des damit zusammenhängenden Stoffwechsels sowie die Therapie von damit im Zusammenhang stehenden Erkrankungen.

Insbesondere betrifft die vorliegende Erfindung ein Verfahren zur Herstellung von Glyceriden der 3-Hydroxybuttersäure sowie die auf diese Weise erhältlichen bzw. hergestellten Reaktionsprodukte (d. h. Glyceride der 3-Hydroxybuttersäure) und deren Verwendung, insbesondere in pharmazeutischen Zusammensetzungen, wie Arzneimitteln oder Medikamenten, oder in Nahrungsmittel- und/oder Lebensmittelerzeugnissen, sowie deren weitere Anwendungen bzw. Verwendungen.

Des Weiteren betrifft die vorliegende Erfindung pharmazeutische Zusammensetzungen, insbesondere Arzneimittel oder Medikamente, welche die nach dem erfindungsgemäßen Herstellungsverfahren erhältlichen bzw. hergestellten Reaktionsprodukte (d. h. Glyceride der 3-Hydroxybuttersäure) umfassen, sowie deren Anwendungen bzw. Verwendungen.

Schließlich betrifft die vorliegende Erfindung Nahrungsmittel- und/oder Lebensmittelerzeugnisse, insbesondere Nahrungsergänzungsmittel, funktionelle Lebensmittel (*Functional Food*)*, Novel Food,* Lebensmittelzusatzstoffe, Nahrungszusätze, diätetische Lebensmittel, Power-Snacks, Appetitzügler und Kraft- und/oder Ausdauersport-Supplements, welche die nach dem erfindungsgemäßen Herstellungsverfahren erhältlichen bzw. hergestellten Reaktionsprodukte (d. h. Glyceride der 3-Hydroxybuttersäure) umfassen, sowie deren Anwendungen bzw. Verwendungen.

Im menschlichen Energiestoffwechsel ist Glucose der kurzfristig zur Verfügung stehende Energieträger, welcher in den Mitochondrien unter Freisetzung von Wasser und Kohlendioxid zu Energie verstoffwechselt wird. Bereits durch die Schlafperiode während der Nacht sind aber die Glycogenspeicher der Leber geleert. Jedoch benötigen vor allem das menschliche zentrale Nervensystem (ZNS) und das Herz eine permanente Energieversorgung.

Die physiologische Alternative zu Glucose, welche vor allem dem zentralen Nervensystem zur Verfügung steht, sind die sogenannten Ketokörper (synonym auch als Ketonkörper bezeichnet oder Englisch auch als *"Keton Bodies"* bezeichnet).

Der Begriff der Ketokörper ist insbesondere eine Sammelbezeichnung für drei Verbindungen, welche vor allem in katabolen Stoffwechsellagen (wie z. B. bei Hunger, Reduktionsdiät oder kohlenhydratarmer Ernährung) gebildet werden und unter Umständen zu einer Ketose führen. Unter den Begriff der Ketokörper fasst man insbesondere die drei Verbindungen Acetoacetat (synonym auch Acetacetat genannt) und Aceton sowie 3-Hydroxybuttersäure (nachfolgend synonym auch als beta-Hydroxybuttersäure oder BHB oder 3-BHB bezeichnet) bzw. deren Salz (d. h. 3-Hydroxybutyrat oder beta-Hydroxybutyrat) zusammen, wobei letztere Verbindung die bedeutendste der drei vorgenannten Verbindungen ist. 3-Hydroxybuttersäure bzw. deren Salz kommt physiologisch als (R)-Enantiomer vor, d. h. als (R)-3-Hydroxybuttersäure (synonym auch (3R)-3-Hydroxybuttersäure genannt, um das Chiralitätszentrum in 3-Position hervorzuheben) bzw. deren Salz.

Diese Ketokörper werden auch in großer Zahl beim Fasten oder Hungern physiologisch aus im Körper eingelagerten Lipiden durch Lipolyse bereitgestellt und ersetzen den Energieträger Glucose fast vollständig.

Die Ketokörper werden in der Leber aus Acetyl-Coenzym A (= Acetyl-CoA) gebildet, welches aus der beta-Oxidation stammt; sie stellen eine transportable Form des Acetyl-Coenzyms A im menschliche Körper dar. Zur Verwertung der Ketokörper müssen sich Gehirn und Muskeln aber zunächst umstellen, indem sie Enzyme exprimieren, welche zur Rückwandlung von Ketokörpern in Acetyl-Coenzym A benötigt werden. Insbesondere in Hungerzeiten tragen die Ketokörper einen beträchtlichen Anteil zur Energiegewinnung bei. So ist es beispielsweise dem Gehirn nach einiger Zeit möglich, mit nur einem Drittel der Tagesmenge an Glucose auszukommen.

Physiologisch erfolgt die Synthese der Ketokörper aus zwei Molekülen aktivierter Essigsäure in Form von Acetyl-Coenzym A, dem normalen Zwischenprodukt des Fettsäureabbaus, wobei zunächst mit Hilfe der Acetyl-Coenzym A-Acetyltransferase das Acetoacetyl-Coenzym A gebildet wird, welches unter Verwendung einer weiteren Acetyl-Coenzym A-Einheit und des Enzyms HMG-CoA-Synthase zum Zwischenprodukt 3-Hydroxy-3-methyl-glutaryl-CoA (HMG-CoA) verlängert wird, wobei schließlich die HMG-CoA-Lyase das Acetoacetat abspaltet. Diese drei Schritte finden ausschließlich in den Mitochondrien der Leber statt (Lynenzyklus), wobei 3-Hydroxybutyrat schließlich im Zytosol durch die D-beta-Hydroxybutyrat-Dehydrogenase entsteht. HMG-CoA ist außerdem ein Endprodukt beim Abbau der Aminosäure Leucin, während Acetoacetat beim Abbau der Aminosäuren Phenylalanin und Tyrosin entsteht.

Durch spontane Decarboxylierung entsteht aus Acetoacetat Aceton; es ist gelegentlich im Atem von Diabetikern und Diäthaltenden wahrzunehmen. Es kann vom Körper nicht weiterverwendet werden. Der Anteil von Aceton an den Ketokörpern ist allerdings gering.

Acetoacetat wird also reduktiv in die physiologisch relevante Form der 3-Hydroxybuttersäure bzw. des 3-Hydroxybutyrats überführt, kann aber auch unter Kohlenstoffdioxidfreisetzung in das physiologisch unbrauchbare Aceton zerfallen, was bei einer schweren Ketose, einer Ketoazidose (z. B. bei Diabetes mellitus Typ 1-Patienten ohne Insulinsubstitution), im Urin und in der Ausatemluft nachweisbar und olfaktorisch wahrnehmbar ist.

3-Hydroxybuttersäure wird derzeit im Bereich des Kraftsports als Natrium-, Magnesium- oder Calcium-Salz eingesetzt und in den Handel gebracht.

Jedoch ist 3-Hydroxybuttersäure für den Menschen evolutionär nicht oder in nur sehr geringer Menge bekannt, da Pflanzen keine 3-Hydroxybuttersäure produzieren und 3-Hydroxybuttersäure im tierischen Organismus nur bei toten ausgezehrten Tieren in der Ketose vorkommt, so dass 3-Hydroxybuttersäure bei peroraler Verabreichung Brechreiz auslöst. 3-Hydroxybuttersäure in Form der freien Säure sowie deren Salzen schmeckt zudem stark bitter und kann schweres Erbrechen und Übelkeit hervorrufen.

Zudem können Patienten, vor allem Neugeborene, aber auch Erwachsene größere Mengen an Salzen der 3-Hydroxybuttersäure nicht permanent verkraften, da diese Verbindungen nierenschädigend wirken können.

Außerdem ist die Plasmahalbwertszeit von 3-Hydroxybuttersäure und deren Salzen derart gering, dass selbst bei Einnahme von mehreren Gramm die Ketose nur für ca. drei bis vier Stunden vorhält, d. h. Patienten können insbesondere während der Nacht daher nicht von einer Therapie mit 3-Hydroxybuttersäure oder deren Salzen kontinuierlich profitieren. Bei Stoffwechselerkrankungen kann dies zu lebensbedrohlichen Situationen führen.

Daher werden im Fall der Therapie derartiger Stoffwechselerkrankungen heute sogenannte mittelkettige Triglyceride, sogenannte MCTs, für die ketogene Therapie eingesetzt, d. h. es wird die metabolische Umwandlung von Capron-, Capryl- und Caprinsäure (d. h. von gesättigten linearen C₆-, C₈- und C₁₀-Fettsäuren) aus den korrespondierenden Triglyceriden beabsichtigt.

Grundsätzlich stellt aber aus pharmazeutischer und klinischer Hinsicht 3-Hydroxybuttersäure demgegenüber ein wirksameres pharmazeutisch-pharmakologisches Zielmolekül, welches nach den Erkenntnissen des Standes der Technik prinzipiell für die Therapie einer Vielzahl von Erkrankungen zum Einsatz kommen könnte, aber aufgrund seiner mangelnden physiologischen Kompatibilität dort nicht zum Einsatz kommen kann (z. B. bei Erkrankungen im Zusammenhang mit einer Störung des Energiestoffwechsels, insbesondere Ketokörperstoffwechsels, oder neurodegenerativen Erkrankungen wie Demenz, Morbus Alzheimer, Morbus Parkinson etc., Fettstoffwechselerkrankungen usw.).

Die nachfolgende Tabelle veranschaulicht rein beispielhaft, aber keinesfalls beschränkend potentielle Therapiemöglichkeiten bzw. mögliche Indikationen für den Wirkstoff 3-Hydroxybuttersäure.

| **Indikation** | **Therapeutischer Effekt** |
|---|---|
| Schädel-Hirn-Trauma | Unter BHB sinkt die Apoptose und Nekrose-Rate von Nervenzellen. |
| Schlaganfall | Unter BHB sinkt die Apoptose und Nekrose-Rate von Nervenzellen. |
| Refeeding-Syndrom | Bei Anorexie, Absetzen enteraler oder parenteraler Ernährung und nach langen Hungerperioden kann der Konsum von Stärke oder Glucose zum Tod führen (siehe auch WHO-Schema Erdnusspaste). BHB kann hier als Therapeutikum zum schnelleren Erlangen einer normalen Nahrungsaufnahme eingesetzt werden. |
| Appetitzügler | BHB unterdrückt im Zentralnervensystem (ZNS) das Hungergefühl. |
| Epilepsie | Herkömmliche ketogene Diät zur signifikanten Reduzierung der Häufigkeit von Krampfanfällen hat extrem schlechte Patienten-Verträglichkeit. BHB bietet hier eine unmittelbar wirksame Alternative. |
| Morbus Alzheimer, Demenz | Unter BHB zeigen Patienten eine bessere kognitive Leistung. BHB ist auch für die Prävention von neurodegenerativen Erkrankungen wirksam. |
| Störungen der Fettsäureoxidation (z. B. Electron-Transfer-Protein Defekt) | Ausgleich eines Nährstoffmangels bei Defekt im Energiestoffwechsel. |

Daher ist es aus pharmazeutischer und klinischer Hinsicht wünschenswert, wirksame Präkursoren oder Metabolite auffinden zu können, welche physiologisch einen direkten oder indirekten Zugang zu 3-Hydroxybuttersäure oder deren Salzen ermöglichen, insbesondere im physiologischen Stoffwechsel des menschlichen oder tierischen Körpers.

Folglich hat es im Stand der Technik nicht an Versuchen gefehlt, physiologisch geeignete Präkursoren oder Metaboliten für 3-Hydroxybuttersäure bzw. deren Salze aufzufinden. Bislang wurden im Stand der Technik jedoch keine effizienten diesbezüglichen Verbindungen aufgefunden. Auch ist ein diesbezüglicher Zugang zu solchen Verbindungen nach dem Stand der Technik bislang nicht bzw. nicht ohne Weiteres möglich.

Die WO 2019/002828 A1 betrifft eine Verbindung zur Verwendung bei der Reduktion von Leberfett eines Patienten, wobei die Verbindung ausgewählt ist aus: (i) (R)-3-Hydroxybutyrat; (ii) einem Ester von (R)-3-Hydroxybutyrat; und (iii) einem Oligomer erhältlich durch Polymerisieren von (R)-3-Hydroxybutyrat-Einheiten; oder einem pharmazeutisch verträglichen Salz oder Solvat davon, wobei die Verbindung besonders nützlich bei Patienten ist, die an einer Fettleber leiden, wobei die Verbindung zur Behandlung von nichtalkoholischer Fettlebererkrankung (NAFLD), nichtalkoholischer Steatohepatitis (NASH), alkoholischer Steatohepatitis (ASH) oder nichtalkoholischer Fettleber (NAFL) verwendet werden kann.

Darüber hinaus betrifft WO 02/06368 A2 ein Polyurethan, welches ein Reaktionsprodukt aus mindestens einem Isocyanat enthaltenden Material mit mindestens zwei Isocyanatgruppen und mindestens einer Verbindung mit mindestens zwei Wasserstoffatomen ist, welche mit dem Isocyanat reagieren können, wobei die Verbindung mit den mindestens zwei Wasserstoffatomen ein Hydroxyalkanoat enthält, welches vorzugsweise ein thermisch zersetzbares oder ein biologisch abbaubares Polyhydroxyalkanoat ist, wobei das Polyurethan in einer Reihe von Anwendungen verwendet werden kann und wobei die Polyurethane vorzugsweise biologisch abbaubar sind und leicht rezykliert werden können.

Die WO 2008/005818 A1 betrifft alternative Quellen für Ketokörper zur Verringerung oder Beseitigung der Symptome von Parkinson, amyotropher Lateralsklerose (ALS bzw. auch Lou Gehrig genannt), Alzheimer, Huntington, Epilepsie und anderer Krankheiten oder Störungen, welche durch einen gestörten Glukosestoffwechsel gekennzeichnet sind, wobei die alternativen Quellen für Ketokörper Mono-, Di- oder Triglyceridester von Acetoacetat und Gemischen davon bzw. Mono-, Di- oder Triglyceridester von 3-Hydroxybutyrat und deren Mischungen umfassen, wobei die Glyceridester oral als Nahrungsergänzungsmittel oder in einer Nährstoffzusammensetzung verabreicht werden können.

Weiterhin betrifft die WO 2013/150153 Ketokörper und Ketokörperester zur oralen Verabreichung zu Zwecken der Verbesserung oder zum Erhalt der Muskelkraft, wobei bestimmte Ester von Hydroxybutyratmonomeren organoleptisch akzeptabel sind und zu einer hohen Aufnahme vom Darm ins Blut führen sollen, wodurch ein schneller Anstieg der Hydroxybutyratkonzentration im Blut und eine physiologische Reaktion einschließlich einer verbesserten Leistungsabgabe während des Trainings ermöglicht werden soll, sowie darüber hinaus Zusammensetzungen, welche die Ketokörper oder Ketokörperester enthalten.

Die WO 2006/034361 A1 betrifft ein Verfahren, um einem Patienten mit Parkinson oder anderen ZNS-Störungen, welche aus einem Dopaminmangel im Gehirn resultieren, eine akute symptomatische Linderung zu verschaffen, wobei das Verfahren die Verabreichung einer ausreichenden Menge eines ketogenen Materials an den Patienten umfasst, um bei dem Patienten eine Ketose zu erzeugen, die ausreicht, um einen therapeutischen Nutzen bei derartigen neurologischen Störungen zu bieten, wobei bevorzugte Materialien, welche eine Ketose erzeugen, derart sind, dass die Gesamtkonzentration von Acetoacetat und (R)-3-Hydroxybutyrat im Blut des Patienten auf zwischen 0,1 und 30 mM erhöht wird.

Darüber hinaus betrifft die WO 95/09144 A1 Zusammensetzungen, welche als parenterale Nährstoffe nützlich sind, wobei diese Verbindungen wasserlösliche Glycerinester der 3-Hydroxybuttersäure sind, wobei die Zusammensetzung als Ersatz für Glykose bei der intravenösen Nahrungszufuhr nützlich sein sollen.

Darüber hinaus betrifft die WO 2018/195421 A1 einen Nährstoff, welcher oral eine therapeutisch wirksame Menge eines Ketokörpers abgibt, welcher ausgewählt ist aus der Gruppe von D-β-Hydroxybutyrat (D-βOHB), Acetoacetat (AcAc), Aceton und einer ihrer Isoformen, in einem Trägervehikel, um Hypersensitivitäten und allergische Störungen zu verhindern und behandeln.

Weiterhin betrifft die WO 2018/132189 A1 ein Verfahren zur Behandlung von leichten bis mittelschweren geschlossenen traumatischen Hirnverletzungen (TBI) und leichten bis mittelschwerer TBI aufgrund eines chirurgischen Eingriffs unter Verwendung von 3-Hydroxybutyratglyceriden.

Dokument WO 2018/118369 betrifft ein Verfahren zur Behandlung der Verringerung von Migränesymptomen und/oder zur Behandlung und/oder Prophylaxe unter Verwendung von 3-Hydroxybutyratglyceriden.

Weiterhin betrifft die EP 0 736 256 A1 das allgemeine Gebiet der enteralen und parenteralen Ernährungsformulierungen, in denen enantiomerenangereicherte Energiesubstrate verwendet werden, um eine hohe Kaloriendichte und eine niedrige Osmolarität bereitzustellen, wobei insbesondere monoisomere Glyceryl-tri-(chirale Alkanoate), anomere Gemische von alpha,beta-D-Glucopyranosyl-alkanoaten, monoisomere chirale Alkansäureester und anomere chirale Alkansäureester zur Anwendung kommen. Ein Verfahren zu deren Herstellung sowie deren Verwendung in der enteralen oder parenteralen Ernährung, bei Stoffwechselstörungen, Traumata und Unterernährung werden ebenfalls beschrieben.

Das der vorliegenden Erfindung zugrundeliegende Problem liegt also in der Bereitstellung eines effizienten Herstellungsverfahrens von physiologisch geeigneten bzw. physiologisch kompatiblen Präkursoren und/oder Metaboliten von 3-Hydroxybuttersäure (d. h. beta-Hydroxybuttersäure bzw. BHB bzw. 3-BHB) oder deren Salzen.

Ein solches Verfahren soll insbesondere die betreffenden BHB-Präkursoren und/oder BHB-Metaboliten in effizienter Weise zugänglich machen, insbesondere auch in größeren Mengen und ohne nennenswerte Mengen an toxischen Nebenprodukten.

In vollkommen überraschender Weise hat die Anmelderin nunmehr herausgefunden, dass Glyceride der 3-Hydroxybuttersäure (beta-Hydroxybuttersäure bzw. BHB bzw. 3-BHB) einen effizienten und physiologisch wirksamen bzw. physiologisch kompatiblen Präkursor und/oder Metaboliten für den Ketokörper 3-Hydroxybuttersäure bzw. deren Salze darstellen und hat in diesem Zusammenhang ein effizientes Herstellungsverfahren für diese Verbindungen auffinden bzw. entwickeln können, welches einen direkten und wirksamen, insbesondere ökonomischen wie auch großtechnisch umsetzbaren Zugang zu diesen Verbindungen ermöglicht.

Zur Lösung des zuvor geschilderten Problems schlägt die vorliegende Erfindung daher - gemäß einem **ersten** Aspekt der vorliegenden Erfindung - ein Verfahren zur Herstellung von Glyceriden der 3-Hydroxybuttersäure (beta-Hydroxybuttersäure bzw. BHB bzw. 3-BHB) gemäß Anspruch 1 vor; weitere, insbesondere besondere und/oder vorteilhafte Ausgestaltungen des erfindungsgemäßen Verfahrens sind Gegenstand der diesbezüglichen Verfahrensunteransprüche.

Weiterhin betrifft die vorliegende Erfindung - gemäß einem **zweiten** Aspekt der vorliegenden Erfindung - ein nach dem erfindungsgemäßen Verfahren erhältliches Reaktionsprodukt gemäß dem diesbezüglichen unabhängigen Anspruch (Anspruch 6) bzw. ein diesbezüglich erhältliches Gemisch von Mono-, Di- und Triglyceriden der 3-Hydroxybuttersäure gemäß dem diesbezüglich unabhängigen Anspruch (Anspruch 7); weitere, insbesondere besondere und/oder vorteilhafte Ausgestaltungen dieses Erfindungsaspekts sind Gegenstand der diesbezüglichen Unteransprüche.

Gleichermaßen betrifft die vorliegende Erfindung - gemäß einem **dritten** Aspekt der vorliegenden Erfindung - eine pharmazeutische Zusammensetzung, insbesondere Arzneimittel oder Medikament, gemäß dem diesbezüglichen unabhängigen Anspruch (Anspruch 10); weitere, insbesondere besondere und/oder vorteilhafte Ausgestaltungen dieses Erfindungsaspekts sind Gegenstand des diesbezüglichen Unteranspruchs.

Darüber hinaus betrifft die vorliegende Erfindung - gemäß einem **vierten** Aspekt der vorliegenden Erfindung - ein erfindungsgemäßes Reaktionsprodukt bzw. ein erfindungsgemäßes Gemisch zur prophylaktischen und/oder therapeutischen Behandlung bzw. zur Verwendung bei der prophylaktischen und/oder therapeutischen Behandlung von Erkrankungen des menschlichen oder tierischen Körpers gemäß dem diesbezüglichen unabhängigen Anspruch (Anspruch 12).

Darüber hinaus betrifft die vorliegende Erfindung - gemäß einem **fünften** Aspekt der vorliegenden Erfindung - ein erfindungsgemäßes Reaktionsprodukt bzw. ein erfindungsgemäßes Gemisch zur Anwendung bei katabolen Stoffwechsellagen, insbesondere Hunger, Diäten oder kohlenhydratamer Ernährung gemäß dem diesbezüglichen unabhängigen Anspruch (Anspruch 13).

Des Weiteren betrifft die vorliegende Erfindung - gemäß einem **sechsten** Aspekt der vorliegenden Erfindung - ein Nahrungsmittel und/oder Lebensmittelerzeugnis gemäß dem diesbezüglichen unabhängigen Anspruch (Anspruch 15); weitere, insbesondere besondere und/oder vorteilhafte Ausgestaltungen des erfindungsgemäßen Nahrungsmittel- und/oder Lebensmittelerzeugnisses sind Gegenstand des diesbezüglichen Unteranspruchs. Schließlich betrifft die vorliegende Erfindung - gemäß einem **siebten** Aspekt der vorliegenden Erfindung - die Verwendung eines erfindungsgemäßen Reaktionsprodukts bzw. eines erfindungsgemäßen Gemischs in einem Nahrungsmittel- und/oder Lebensmittelerzeugnis gemäß dem diesbezüglichen unabhängigen Anspruch (Anspruch 16).

Es versteht sich bei den nachfolgenden Ausführungen von selbst, dass Ausgestaltungen, Ausführungsformen, Vorteile und dergleichen, welche nachfolgend zu Zwecken der Vermeidung von Wiederholungen nur zu einem Erfindungsaspekt ausgeführt sind, selbstverständlich auch in Bezug auf die übrigen Erfindungsaspekte entsprechend gelten, ohne dass dies einer gesonderten Erwähnung bedarf.

Des Weiteren versteht es sich von selbst, dass einzelne Aspekte und Ausführungsformen der vorliegenden Erfindung auch in beliebiger Kombination mit anderen Aspekten und Ausführungsformen der vorliegenden Erfindung als offenbart gelten und insbesondere auch eine beliebige Kombination von Merkmalen und Ausführungsformen, wie sie sich aus den Rückbezügen aller Patentansprüche ergibt, umfangreich als offenbart gilt, und zwar im Hinblick auf alle sich ergebenden Kombinationsmöglichkeiten.

Bei allen nachstehend genannten relativen bzw. prozentualen gewichtsbezogenen Angaben, insbesondere relativen Mengen- oder Gewichtsangaben, ist weiterhin zu beachten, dass diese im Rahmen der vorliegenden Erfindung vom Fachmann derart auszuwählen sind, dass sie sich in der Summe unter Einbeziehung aller Komponenten bzw. Inhaltsstoffe, insbesondere wie nachfolgend definiert, stets zu 100 % bzw. 100 Gew.-% ergänzen bzw. addieren; dies versteht sich aber für den Fachmann von selbst.

Im Übrigen gilt, dass der Fachmann - anwendungsbezogen oder aber einzelfallbedingt - von den nachfolgend angeführten Bereichsangaben erforderlichenfalls abweichen kann, ohne dass er den Rahmen der vorliegenden Erfindung verlässt.

Zudem gilt, dass alle im Folgenden genannten Werte- bzw. Parameterangaben oder dergleichen grundsätzlich mit genormten bzw. standardisierten oder explizit angegebenen Bestimmungsverfahren oder andernfalls mit dem Fachmann auf diesem Gebiet an sich geläufigen Bestimmungs- bzw. Messmethoden ermittelt bzw. bestimmt werden können.

Dies vorausgeschickt, wird die vorliegende Erfindung nunmehr nachfolgend im Detail erläutert.

Gegenstand der vorliegenden Erfindung - gemäß einem **ersten** Aspekt der vorliegenden Erfindung - ist somit ein
Verfahren zur Herstellung von Glyceriden der 3-Hydroxybuttersäure (beta-Hydroxybuttersäure, BHB bzw. 3-BHB),
wobei mindestens eine Verbindung der allgemeinen Formel (I)

   CH₃-CH(OH)-CH₂-C(O)OR¹ (I)
wobei in der allgemeinen Formel (I) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt,
mit Glycerin (1 ,2,3-Propantriol) der Formel (II)

   CH₂(OH) - CH(OH) - CH₂(OH) (II)
umgesetzt wird,
wobei die Umsetzung in Gegenwart eines Enzyms als Katalysator durchgeführt wird und
wobei die Umsetzung in Abwesenheit von Lösemitteln und/oder ohne jedwedes Lösemittel durchgeführt wird,
so dass als Reaktionsprodukt ein Gemisch von Mono-, Di- und Triglyceriden der 3-Hydroxybuttersäure der allgemeinen Formel (III)

   CH₂(OR²)-CH(OR³)-CH₂(OR⁴) (III)
erhalten wird, wobei in der allgemeinen Formel (III) die Reste R², R³ und R⁴, jeweils unabhängig voneinander, Wasserstoff oder einen Rest der Formel CH₃- CH(OH) - CH₂-C(O)- darstellen, jedoch mit der Maßgabe, dass mindestens ein Rest R², R³ und R⁴ keinen Wasserstoff darstellt,
wobei das Gemisch Monoglyceride der 3-Hydroxybuttersäure (3-BHB-MG), Diglyceride der 3-Hydroxybuttersäure (3-BHB-DG) und Triglyceride der 3-Hydroxybuttersäure (3-BHB-TG) in einem gewichtsbezogenen Verhältnis von 3-BHB-MG/3-BHB-DG/3-BHB-TG im Bereich von 10-80 / 10-70 / 0,1-20 umfasst und
wobei bei der Umsetzung gleichzeitig die Verbindung gemäß der allgemeinen Formel (IV)

   R¹-OH (IV)
gebildet wird, wobei in der allgemeinen Formel (IV) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt, wobei die Verbindung gemäß der allgemeinen Formel (IV) der Umsetzung kontinuierlich entzogen wird.

Wie zuvor ausgeführt, hat die Anmelderin nämlich vollkommen überraschend herausgefunden, dass die auf diese Weise hergestellten Glyceride der 3-Hydroxybuttersäure (nachfolgend auch kurz nur als "BHB-Glyceride"/"3-BHB-Glyceride" oder "BHB-Ester"/"3-BHB-Ester" bezeichnet) effiziente, da physiologisch verträgliche Präkursoren und/oder Metabolite der 3-Hydroxybuttersäure bzw. deren Salzen darstellen, welche pharmazeutisch bzw. klinisch auch in größeren Mengen zum Einsatz kommen können, da sie physiologisch kompatibel sind.

Die vorgenannten Glyceride der 3-Hydroxybuttersäure, welche durch das erfindungsgemäße Herstellungsverfahren erstmals in effizienter Weise zugänglich sind, stellen eine physiologisch und pharmakologisch relevante Alternative zu der freien 3-Hydroxybuttersäure bzw. deren Salzen dar.

Die Herstellung von Glyceriden der 3-Hydroxybuttersäure mittels herkömmlicher organischer Synthese ist komplex und aufwendig, da 3-Hydroxybuttersäure verstärkt zur Polymerisation und anderen unerwünschten Nebenreaktionen (z. B. Wasserabspaltung, Zersetzung etc.) neigt. Im Rahmen der vorliegenden Erfindung konnte erstmals ein effizient arbeitendes Herstellungsverfahren bereitgestellt werden, mit welchem sich Glyceride der 3-Hydroxybuttersäure ohne unerwünschte Nebenreaktionen herstellen lassen, insbesondere einstufig.

Das erfindungsgemäße Verfahren ermöglicht somit erstmalig die Bereitstellung untoxischer Ester der 3-Hydroxybuttersäure aus an sich bekannten, kommerziell verfügbaren und vor allem physiologisch unbedenklichen Komponenten bzw. Edukten (Ausgangsverbindungen). Die resultierenden Glyceride können physiologisch, insbesondere im Magen und/oder im Darm, aufgespalten werden und das Zielmolekül "3-Hydroxybuttersäure" bzw. deren Salze als Wirkstoff bzw. Wirkkomponente freisetzen bzw. generieren.

Darüber hinaus weisen die vorgenannten Glyceride der 3-Hydroxybuttersäure auch einen akzeptablen Geschmack auf, um eine Kompatibilität auch bei oraler Verabreichung größerer Mengen über einen längeren Zeitraum zu gewährleisten (z. B. Verabreichung von 50 g Tagesdosis oder mehr).

Gleichermaßen ermöglicht es das erfindungsgemäße Herstellungsverfahren die Glyceride der 3-Hydroxybuttersäure frei von toxischen Verunreinigungen bereitzustellen.

Darüber hinaus kann bei entsprechenden Ausgangsmaterialien die Herstellung auch enantioselektiv durchgeführt werden. So ermöglicht es beispielsweise das erfindungsgemäße Herstellungsverfahren, die biologisch relevante Form, d. h. das (R)-Enantiomer anzureichern, insbesondere durch Enzymkatalyse, um bei oraler Verabreichung das renale System von Patienten nicht zu belasten (d. h. Elimination über die Nieren). Grundsätzlich ist es aber auch möglich und kann es unter bestimmten Voraussetzungen zweckdienlich sein, das (S)-Enantiomer anzureichern.

Darüber hinaus ist das erfindungsgemäße Herstellungsverfahren, einschließlich optionaler Weiterverarbeitungs- bzw. Aufreinigungsverfahrensschritte, wirtschaftlich bzw. ökonomisch betreibbar und auch großtechnisch umsetzbar.

Insbesondere verwendet das erfindungsgemäße Herstellungsverfahren kommerziell verfügbare Edukte und ermöglicht darüber hinaus eine relativ einfache Verfahrensführung auch bei großtechnischer Umsetzung.

Im Gegensatz zu herkömmlichen Herstellungsverfahren des Standes der Technik, welche über komplexe Ausgangsverbindungen und über entsprechende Schutzgruppenchemie verlaufen, wie z. B. Diketene (z. B. WO 95/09144 A1 oder WO 90/02549 A1), oder komplexe mehrstufige Synthesen (z. B. US 6 306 828 B1) anwenden, kommt das erfindungsgemäße Herstellungsverfahren ohne derartige komplexe Edukte aus und verläuft nur einstufig. Dennoch werden im Rahmen des erfindungsgemäßen Herstellungsverfahrens exzellente Ausbeuten erzielt, wobei in gleicher Weise die Bildung von Nebenprodukten minimiert bzw. vermieden wird.

Darüber hinaus ist das erfindungsgemäßen Verfahren einfach und wirtschaftlich. Insbesondere wird das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*); folglich sind die erhaltenen Reaktionsprodukte nicht mit Lösemittel verunreinigt und es muss kein Lösemittel nach Durchführung des Verfahrens bzw. der Reaktion aufwendig und energieträchtig entfernt und entsorgt bzw. rezykliert werden. Es werden zudem auch keine toxischen Nebenprodukte gebildet.

Das erfindungsgemäße Herstellungsverfahren führt zu einem Gemisch verschiedener Glyceride der 3-Hydroxybuttersäure, d. h. von Mono-, Di- und Triglyceriden der 3-Hydroxybuttersäure. Das resultierende Rohreaktionsprodukt bzw. Rohglyceridgemisch kann mit an sich bekannten Methoden aufgereinigt werden, insbesondere von gegebenenfalls noch vorhandenen Edukten und/oder gegebenenfalls vorhandenen Nebenprodukten befreit werden, und - sofern gewünscht- mit ebenfalls an sich bekannten Methoden aufgespalten werden, insbesondere destillativ und/oder chromatographisch (z. B. Fraktionierung in die einzelnen Glyceride, d. h. Mono-, Di- und Triglyceride der 3-Hydroxybuttersäure, oder aber Fraktionierung in Fraktionen mit angereichertem und abgereichertem Anteil einzelner Glyceride oder aber Fraktionierung in ein Mono- und Diglycerid-Gemisch einerseits und das Triglycerid andererseits oder aber Fraktionierung in das Monoglycerid einerseits und ein Di- und Triglycerid-Gemisch andererseits etc.).

Gemäß einer besonderen Ausführungsform der vorliegenden Erfindung kann die Verbindung der allgemeinen Formel (I) entweder in racemischer Form oder aber in Form des (R)-Enantiomers eingesetzt werden. Die (R)-Konfiguration bezieht sich auf das chirale Kohlenstoffatom in 3-Position der Verbindung der allgemeinen Formel (I).

Erfindungsgemäß bevorzugt ist es, wenn in der allgemeinen Formel (I) der Rest R¹ Ethyl darstellt.

Mit anderen Worten ist es erfindungsgemäß bevorzugt, dass als Verbindung der allgemeinen Formel (I) 3-Hydroxybuttersäureethylester (Ethyl-3-hydroxybutyrat) der Formel CH₃-CH(OH)-CH₂-C(O)OC₂H₅ eingesetzt wird.

Dies ermöglicht eine besonders effiziente Verfahrensführung und hohe Ausbeuten mit minimierter bzw. unterdrückter Nebenproduktbildung. Zudem ist der 3-Hydroxybuttersäureethylester auch in großen Mengen kommerziell verfügbar und zudem ökonomisch effizienter als die freie Säure (d. h. 3-Hydroxybuttersäure) umsetzbar. Insbesondere kann der 3-Hydroxybuttersäureethylester als Ausgangsverbindung großtechnisch z. B. durch Claisen-Kondensation von Ethylacetat gewonnen werden.

Bei dem erfindungsgemäßen Verfahren wird die Umsetzung in Abwesenheit von Lösemitteln und/oder ohne jedwedes Lösemittel durchgeführt. D. h. die Umsetzung wird also als Reaktion in Masse bzw. als Reaktion in Substanz bzw. als sogenannte *Bulk Reaction* durchgeführt. Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Gemäß der vorliegenden Erfindung wird die Umsetzung in Gegenwart eines Enzyms als Katalysator durchgeführt. Dabei ist es bevorzugt, wenn der Katalysator nach der Umsetzung rezykliert wird.

Wie zuvor ausgeführt, wird im Rahmen des erfindungsgemäßen Herstellungsverfahrens die Umsetzung in Gegenwart eines Enzyms als Katalysator durchgeführt.

Dabei kann das Enzym insbesondere ausgewählt sein aus Synthetasen (Ligasen), Katalasen, Esterasen, Lipasen und deren Kombinationen. Erfindungsgemäß werden als Synthetasen (synonym Ligasen) insbesondere Enzyme aus der Klasse der Ligasen bezeichnet; Ligasen sind Enzyme, welche das Verknüpfen zweier oder mehrerer Moleküle durch eine kovalente Bindung katalysieren. Katalasen im Sinne der vorliegenden Erfindung sind insbesondere Enzyme, welche Wasserstoffperoxid zu Sauerstoff und Wasser umzusetzen imstande sind. Der Begriff der Esterasen bezeichnet insbesondere Enzyme, welche imstande sind, Ester hydrolytisch in Alkohol und Säure aufzuspalten (Verseifung); es handelt sich somit insbesondere um Hydrolasen, wobei fettspaltende Esterasen auch als Lipasen bezeichnet werden. Lipasen im Sinne der vorliegenden Erfindung sind insbesondere Enzyme, welche von Lipiden, wie Glyceriden, freie Fettsäuren abzuspalten imstande sind (Lipolyse).

Im Rahmen der vorliegenden Erfindung kann sich das als Katalysator eingesetzte Enzym insbesondere ableiten von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei (Rhizomucor miehei)* und *Thermomyces lanuginosus.*

Gemäß einer besonderen Ausführungsform kann das Enzym in immobilisierter Form, immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt werden.

Wie zuvor im Zusammenhang mit der Verwendung eines Katalysators im Allgemeinen dargelegt, ist es bevorzugt, im Fall der Verwendung eines Enzyms als Katalysator das Enzym nach der Umsetzung zu rezyklieren.

Erfindungsgemäß wird die Umsetzung im Rahmen des erfindungsgemäßen Herstellungsverfahrens in Gegenwart eines Enzyms als Katalysator durchgeführt; dabei ist es bevorzugt, wenn die Umsetzung bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird.

Erfindungsgemäß kann die Menge des eingesetzten Enzyms in weiten Bereichen variieren. Insbesondere kann das Enzym in Mengen, bezogen auf die Gesamtmenge der Ausgangsverbindungen der Formeln (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt werden. Dennoch kann es einzelfallbedingt oder anwendungsbezogen erforderlich sein, von den vorgenannten Mengen abzuweichen, ohne dass der Rahmen der vorliegenden Erfindung verlassen ist.

Erfindungsgemäß kann auch der angewendete Druckbereich in weiten Bereichen variieren. Insbesondere kann bei Umsetzung in Gegenwart eines Enzyms als Katalysator die Umsetzung bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt werden.

Was die Menge an Edukten bzw. Ausgangsverbindungen anbelangt, so kann auch diese in weiten Bereichen variiert werden.

Unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (I), bezogen auf die Hydroxylgruppen des Glycerins der Formel (II), in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird.

Gleichermaßen unter Berücksichtigung von Verfahrensökonomie und Optimierung des Verfahrensablaufs, insbesondere im Hinblick auf die Minimierung von Nebenprodukten, ist es vorteilhaft, wenn die Verbindung der allgemeinen Formel (I) und das Glycerin der Formel (II) in einem Molverhältnis von Verbindung der allgemeinen Formel (I) / Glycerin der Formel (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt werden.

Gemäß einer besonders bevorzugten Ausführungsform der vorliegenden Erfindung betrifft die vorliegende Erfindung ein Verfahren zur Herstellung von Glyceriden der 3-Hydroxybuttersäure (beta-Hydroxybuttersäure, BHB bzw. 3-BHB), insbesondere wie zuvor beschrieben, wobei mindestens eine Verbindung der Formel (la)

CH₃-CH(OH)-CH₂-C(O)OC₂H₅ (Ia)

mit Glycerin (1 ,2,3-Propantriol) der Formel (II)

CH₂(OH) - CH(OH) - CH₂(OH) (II)

umgesetzt wird,
so dass als Reaktionsprodukt ein wie zuvor definiertes Gemisch von Mono-, Di- und Triglyceriden der 3-Hydroxybuttersäure der allgemeinen Formel (III)

CH₂(OR²) - CH(OR³) - CH₂(OR⁴) (III)

erhalten werden, wobei in der allgemeinen Formel (III) die Reste R², R³ und R⁴, jeweils unabhängig voneinander, Wasserstoff oder einen Rest der Formel CH₃-CH(OH) - CH₂ - C(O) - darstellen, jedoch mit der Maßgabe, dass mindestens ein Rest R², R³ und R⁴ keinen Wasserstoff darstellt.

Die Umsetzung wird dabei in Gegenwart eines Enzyms als Katalysator, insbesondere wie zuvor definiert, durchgeführt, insbesondere wie zuvor beschrieben und/oder insbesondere unter den zuvor beschriebenen Bedingungen.

Diese Vorgehensweise führt zu einer besonders guten Verfahrenseffizienz und Verfahrensökonomie, insbesondere verbunden mit hohen Ausbeuten und einer Minimierung der Bildung von Nebenprodukten.

Die erfindungsgemäß besonders bevorzugte Vorgehensweise wird durch das nachfolgende Reaktions- bzw. Syntheseschema veranschaulicht (wobei in diesem Reaktions- bzw. Syntheseschema die Reste R², R³ und R⁴, jeweils unabhängig voneinander, Wasserstoff oder einen Rest der Formel CH₃-CH(OH)-CH₂-C(O) - darstellen, jedoch mit der Maßgabe, dass mindestens ein Rest R², R³ und R⁴ keinen Wasserstoff darstellt):

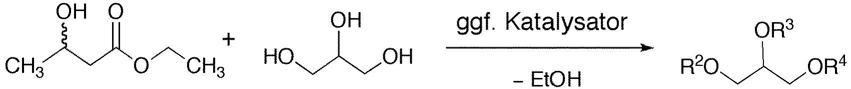

Im Rahmen des erfindungsgemäßen Herstellungsverfahrens wird bei der Umsetzung gleichzeitig die Verbindung gemäß der allgemeinen Formel (IV)

R¹-OH (IV)

gebildet, wobei in der allgemeinen Formel (IV) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt. Mit anderen Worten werden im Rahmen der Umsetzung in Abhängigkeit von der Ausgangsverbindung der allgemeinen Formel (I) Wasser bzw. C₁-C₄-Alkohole gebildet.

In diesem Zusammenhang ist es vorgesehen, dass die Verbindung gemäß der allgemeinen Formel (IV) (d. h. insbesondere Wasser bzw. C₁-C₄-Alkohole) der Umsetzung kontinuierlich entzogen wird, insbesondere mittels vorzugsweise kontinuierlicher destillativer Entfernung. Auf diese Weise wird das Reaktionsgleichgewicht in effizienter Weise auf die Seite der Reaktionsprodukte verlagert. Auch wird auf diese Weise die Bildung von Nebenprodukten minimiert bzw. verhindert.

Gemäß dem erfindungsgemäßen Verfahren wird als Reaktionsprodukt ein Gemisch von Mono-, Di- und Triglyceriden der 3-Hydroxybuttersäure der allgemeinen Formel (III)

CH₂(OR²) - CH(OR³) - CH₂(OR⁴) (III)

erhalten, wobei in der allgemeinen Formel (III) die Reste R², R³ und R⁴, jeweils unabhängig voneinander, Wasserstoff oder einen Rest der Formel CH₃-CH(OH)-CH₂-C(O)- darstellen, jedoch mit der Maßgabe, dass mindestens ein Rest R², R³ und R⁴ keinen Wasserstoff darstellt.

Im Rahmen des erfindungsgemäßen Herstellungsverfahrens werden drei voneinander verschiedene Glyceride der 3-Hydroxybuttersäure der allgemeinen Formel (III), wie zuvor definiert, erhalten.

Gemäß der vorliegenden Erfindung wird im Rahmen des erfindungsgemäßen Herstellungsverfahrens ein Gemisch von Mono-, Di- und Triglyceriden der 3-Hydroxybuttersäure erhalten.

Gemäß der vorliegenden Erfindung wird im Rahmen des erfindungsgemäßen Herstellungsverfahrens als Reaktionsprodukt ein Gemisch von Mono-, Di- und Triglyceriden der 3-Hydroxybuttersäure erhalten. Das Gemisch umfasst Monoglyceride der 3-Hydroxybuttersäure (3-BHB-MG), Diglyceride der 3-Hydroxybuttersäure (3-BHB-DG) und Triglyceride der 3-Hydroxybuttersäure (3-BHB-TG) in einem gewichtsbezogenen Verhältnis von 3-BHB-MG/3-BHB-DG/ 3-BHB-TG im Bereich von 10-80/10-70/0,1-20, insbesondere im Bereich von 20-70 / 20-60 / 0,5-15.

Durch übliche Aufarbeitungs- und/oder Trennverfahren (z. B. Chromatographie, Destillation etc.) lassen sich zudem beliebige Gemische einstellen oder auch die jeweils reinen Glyceride (d. h. reines Monoglycerid der 3-Hydroxybuttersäure oder aber reines Diglycerid der 3-Hydroxybuttersäure oder aber reines Triglycerid der 3-Hydroxybuttersäure) erhalten. Auch Reingemische aus Monoglycerid(en) der 3-Hydroxybuttersäure und Diglycerid(en) der 3-Hydroxybuttersäure lassen sich auf diese Weise erhalten.

Im Rahmen des erfindungsgemäßen Herstellungsverfahrens kann die Zusammensetzung des Reaktionsprodukts, insbesondere der Anteil der verschiedenen Glyceride der 3-Hydroxybuttersäure der allgemeinen Formel (III) mittels der Umsetzungsbedingungen kontrolliert und/oder gesteuert werden. Insbesondere kann dies durch Auswahl der Umsetzungstemperatur (Reaktionstemperatur) und/oder Auswahl des Umsetzungsdrucks (Reaktionsdrucks) und/oder die Auswahl des Katalysators in Bezug auf Art und/oder Menge und/oder Auswahl der Mengen der Ausgangsverbindungen (Edukte) und/oder Vorsehen der Entfernung der Verbindung gemäß der allgemeinen Formel (IV), wie zuvor definiert, erfolgen.

Im Anschluss an die Umsetzung kann das erhaltene Reaktionsprodukt weiteren Aufreinigungs- bzw. Aufarbeitungsschritten unterzogen werden.

In diesem Zusammenhang kann das erhaltene Reaktionsprodukt nach erfolgter Umsetzung fraktioniert werden, insbesondere destillativ fraktioniert werden. Insbesondere kann bei dieser Ausführungsform das Reaktionsprodukt mindestens aufgetrennt werden in eine erste, insbesondere leichtsiedende Fraktion, welche einen hohen Anteil an Mono- und Diglyceriden der 3-Hydroxybuttersäure und einen geringen Anteil an Triglyceriden der 3-Hydroxybuttersäure umfasst, und eine zweite, insbesondere schwersiedende Fraktion, welche einen geringen Anteil an Monoglyceriden der 3-Hydroxybuttersäure und einen hohen Anteil an Di- und Triglyceriden der 3-Hydroxybuttersäure umfasst.

Dabei kann die erste, insbesondere leichtsiedende Fraktion insbesondere ein gewichtsbezogenes Verhältnis von 3-BHB-MG/3-BHB-DG/3-BHB-TG im Bereich von 70-95 / 5-30 / 0,01-2, insbesondere im Bereich von 75-85 / 10-25 / 0-1, umfassen; insbesondere kann die zweite, insbesondere schwersiedende Fraktion ein gewichtsbezogenes Verhältnis von 3-BHB-MG/3-BHB-DG/3-BHB-TG im Bereich von 5-30 / 20-80 / 5-40, insbesondere im Bereich von 5-20 / 40-75 / 10-30, umfassen.

Gemäß einer besonderen Ausführungsform der vorliegenden Erfindung können die nichtumgesetzten Ausgangsverbindungen der Formeln (I) und/oder (II) aus dem Reaktionsprodukt abgetrennt und anschließend rezykliert werden.

Wie zuvor bereits ausgeführt, wird üblicherweise das erfindungsgemäße Verfahren in Abwesenheit von Lösemitteln und/oder ohne jedwedes Lösemittel durchgeführt (d. h. also als Reaktion in Masse bzw. als Reaktion in Substanz bzw. als sogenannte *Bulk Reaction*). Dies hat den Vorteil, dass die erhaltenen Reaktionsprodukte nicht mit Lösemittel verunreinigt sind und kein Lösemittel nach Durchführung des Verfahrens bzw. der Reaktion aufwendig und energieträchtig entfernt und entsorgt bzw. rezykliert werden muss. Überraschenderweise verläuft das Verfahren bzw. die Reaktion dennoch mit hohen Umsätzen und Ausbeuten und zumindest im Wesentlichen ohne signifikante Nebenproduktbildung.

Weiterer Gegenstand - gemäß einem **zweiten** Aspekt der vorliegenden Erfindung - ist das nach dem erfindungsgemäßen Verfahren erhältliche Reaktionsprodukt (d. h. ein (chemisches) Produkt oder Produktgemisch).

Gegenstand gemäß diesem Erfindungsaspekt ist insbesondere ein Gemisch von Mono-, Di- und Triglyceriden der 3-Hydroxybuttersäure der allgemeinen Formel (III)

CH₂(OR²) - CH(OR³) - CH₂(OR⁴) (III)

wobei in der allgemeinen Formel (III) die Reste R², R³ und R⁴, jeweils unabhängig voneinander, Wasserstoff oder einen Rest der Formel CH₃ - CH(OH) - CH₂ - C(O) - darstellen, jedoch mit der Maßgabe, dass mindestens ein Rest R², R³ und R⁴ keinen Wasserstoff darstellt,
wobei das Gemisch Monoglyceride der 3-Hydroxybuttersäure (3-BHB-MG), Diglyceride der 3-Hydroxybuttersäure (3-BHB-DG) und Triglyceride der 3-Hydroxybuttersäure (3-BHB-TG) in einem gewichtsbezogenen Verhältnis von 3-BHB-MG/3-BHB-DG/3-BHB-TG im Bereich von 10-80 / 10-70 / 0,1-20 umfasst.

Gemäß der vorliegenden Erfindung ist Gegenstand der vorliegenden Erfindung ein nach dem erfindungsgemäßen Verfahren erhältliches Gemisch von drei voneinander verschiedenen Glyceriden der 3-Hydroxybuttersäure der allgemeinen Formel (III), wie zuvor definiert.

Gemäß der vorliegenden Erfindung ist Gegenstand der vorliegenden Erfindung ein nach dem erfindungsgemäßen Verfahren erhältliches Gemisch von Mono-, Di- und Triglyceriden der 3-Hydroxybuttersäure.

Gemäß der vorliegenden Erfindung ist Gegenstand der vorliegenden Erfindung ein nach dem erfindungsgemäßen Verfahren erhältliches Gemisch von Mono-, Di- und Triglyceriden der 3-Hydroxybuttersäure. Erfindungsgemäß umfasst das Gemisch Monoglyceride der 3-Hydroxybuttersäure (3-BHB-MG), Diglyceride der 3-Hydroxybuttersäure (3-BHB-DG) und Triglyceride der 3-Hydroxybuttersäure (3-BHB-TG) in einem gewichtsbezogenen Verhältnis von 3-BHB-MG/3-BHB-DG/ 3-BHB-TG im Bereich von 10-80 / 10-70 / 0,1-20, insbesondere im Bereich von 20-70 / 20-60 / 0,5-15.

Gemäß einer wiederum weiteren besonderen Ausführungsform der vorliegenden Erfindung ist Gegenstand der vorliegenden Erfindung ein nach dem erfindungsgemäßen Verfahren erhältliches Gemisch von Mono-, Di- und Triglyceriden der 3-Hydroxybuttersäure, wobei das Gemisch von Mono-, Di- und Triglyceriden der 3-Hydroxybuttersäure die Mono-, Di- und Triglyceride der 3-Hydroxybuttersäure in einem gewichtsbezogenen Verhältnis von 3-BHB-MG/3-BHB-DG/3-BHB-TG im Bereich von 70-95 / 5-30 / 0,01-2, insbesondere im Bereich von 75-85 / 10-25 / 0-1, umfasst. Dieses spezielle Gemisch kann insbesondere durch fraktionierte Destillation als leichtsiedende Fraktion aus dem Reaktionsproduktgemisch erhalten werden.

Weiterhin ist Gegenstand der vorliegenden Erfindung gemäß einer wiederum weiteren besonderen Ausführungsform der vorliegenden Erfindung ein nach dem erfindungsgemäßen Verfahren erhältliches Gemisch, wobei das Gemisch ein Gemisch von Mono-, Di- und Triglyceriden der 3-Hydroxybuttersäure umfasst, wobei das Gemisch von Mono-, Di- und Triglyceriden der 3-Hydroxybuttersäure die Mono-, Di- und Triglyceride der 3-Hydroxybuttersäure in einem gewichtsbezogenen Verhältnis von 3-BHB-MG/ 3-BHB-DG/3-BHB-TG im Bereich von 5-30 / 20-80 / 5-40, insbesondere im Bereich von 5-20 / 40-75 / 10-30, umfasst. Dieses spezielle Gemisch kann insbesondere durch fraktionierte Destillation als schwersiedende Fraktion aus dem Reaktionsproduktgemisch erhalten werden.

Wie zuvor ausgeführt, lassen sich durch übliche Aufarbeitungs- und/oder Trennverfahren (z. B. Chromatographie, Destillation etc.) zudem beliebige Gemische einstellen oder auch die jeweils reinen Glyceride (d. h. reines Monoglycerid der 3-Hydroxybuttersäure oder aber reines Diglycerid der 3-Hydroxybuttersäure oder aber reines Triglycerid der 3-Hydroxybuttersäure) erhalten. Auch Reingemische aus Monoglyceriden der 3-Hydroxybuttersäure und Diglyceriden der 3-Hydroxybuttersäure lassen sich auf diese Weise erhalten.

Das nach dem erfindungsgemäßen Verfahren erhältliche Reaktionsprodukt bzw. Gemisch von Glyceriden weist gegenüber dem Stand der Technik eine Vielzahl von Vorteilen und Besonderheiten auf:
Wie die Anmelderin überraschend herausgefunden hat, eignet sich das nach dem erfindungsgemäßen Verfahren erhältliche Reaktionsprodukt bzw. Glyceridgemisch als Präkursor bzw. Metabolit von 3-Hydroxybuttersäure bzw. deren Salzen, da es einerseits physiologisch, insbesondere im Magen/Darm-Trakt, zu 3-Hydroxybuttersäure bzw. deren Salzen umgesetzt wird und andererseits gleichzeitig eine gute physiologische Kompatibilität bzw. Verträglichkeit aufweist, insbesondere im Hinblick auf Nichttoxizität und akzeptable organoleptische Eigenschaften.

Darüber hinaus ist das erfindungsgemäße Reaktionsprodukt bzw. Glyceridgemisch ohne Weiteres auf synthetischem Wege auch in großtechnischem Maßstab zugänglich bzw. verfügbar, und zwar auch mit der erforderlichen pharmazeutischen bzw. pharmakologischen Qualität.

Zudem kann das erfindungsgemäße Reaktionsprodukt bzw. Glyceridgemisch erforderlichenfalls in enantiomerenreiner bzw. enantiomerenangereicherter Form bereitgestellt werden.

Das erfindungsgemäße Reaktionsprodukt bzw. Glyceridgemisch stellt somit ein effizientes pharmakologisches Wirkstoff-Target im Rahmen einer Ketokörper-Therapie des menschlichen oder tierischen Körpers dar.

Weiterer Gegenstand der vorliegenden Erfindung - gemäß einem **dritten** Aspekt der vorliegenden Erfindung - ist eine pharmazeutische Zusammensetzung, insbesondere ein Arzneimittel oder Medikament, welche(s) ein nach dem erfindungsgemäßen Herstellungsverfahren erhältliches Reaktionsprodukt, wie zuvor definiert, und/oder ein nach dem erfindungsgemäßen Herstellungsverfahren erhältliches Gemisch, wie zuvor definiert, umfasst.

Insbesondere betrifft die vorliegende Erfindung gemäß diesem Erfindungsaspekt eine pharmazeutische Zusammensetzung zur prophylaktischen und/oder therapeutischen Behandlung bzw. zur Verwendung bei der prophylaktischen und/oder therapeutischen Behandlung von Erkrankungen des menschlichen oder tierischen Körpers. Hierbei kann es sich insbesondere um Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glukosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, handeln.

Wiederum weiterer Gegenstand der vorliegenden Erfindung - gemäß einem **vierten** Aspekt der vorliegenden Erfindung - ist ein nach dem erfindungsgemäßen Herstellungsverfahren erhältliches Reaktionsprodukt, wie zuvor definiert, und/oder ein nach dem erfindungsgemäßen Herstellungsverfahren erhältliches Gemisch, wie zuvor definiert, zur prophylaktischen und/oder therapeutischen Behandlung bzw. zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glukosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

Auch ein nach dem erfindungsgemäßen Herstellungsverfahren erhältliches Reaktionsprodukt, wie zuvor definiert, und/oder ein nach dem erfindungsgemäßen Herstellungsverfahren erhältliches Gemisch, wie zuvor definiert, zur prophylaktischen und/oder therapeutischen Behandlung bzw. zur Herstellung eines Arzneimittels zur prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glukosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien, kann vorgesehen sein.

Gleichermaßen weiterer Gegenstand der vorliegenden Erfindung - gemäß einem **fünften** Aspekt der vorliegenden Erfindung - ist ein nach dem erfindungsgemäßen Herstellungsverfahren erhältliches Reaktionsprodukt, wie zuvor definiert, und/oder ein nach dem erfindungsgemäßen Herstellungsverfahren erhältliches Gemisch, wie zuvor definiert, zur prophylaktischen und/oder therapeutischen Behandlung bzw. zur Herstellung eines Arzneimittels zur prophylaktischen und/oder therapeutischen Behandlung bzw. zur Anwendung von/bei katabolen Stoffwechsellagen, wie Hunger, Diäten oder kohlenhydratarmer Ernährung.

Weiterer Gegenstand der vorliegenden Erfindung ist ein erfindungsgemäßes Reaktionsprodukt, wie zuvor definiert, und/oder ein erfindungsgemäßes Gemischs, wie zuvor definiert, zur Verwendung bei der prophylaktischen und/oder therapeutischen Behandlung von katabolen Stoffwechsellagen.

Gleichermaßen weiterer Gegenstand der vorliegenden Erfindung - gemäß einem **sechsten** Aspekt der vorliegenden Erfindung - ist ein Nahrungsmittel- und/oder Lebensmittelerzeugnis, welches ein nach dem erfindungsgemäßen Herstellungsverfahren erhältliches Reaktionsprodukt, wie zuvor definiert, und/oder ein nach dem erfindungsgemäßen Herstellungsverfahren erhältliches Gemisch, wie zuvor definiert, umfasst.

Gemäß einer besonderen Ausführungsform kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Schließlich ist wiederum weiterer Gegenstand der vorliegenden Erfindung - gemäß einem **siebten** Aspekt der vorliegenden Erfindung - die Verwendung eines nach dem erfindungsgemäßen Herstellungsverfahren erhältlichen Reaktionsprodukts, wie zuvor definiert, und/oder eines nach dem erfindungsgemäßen Herstellungsverfahren erhältlichen Gemischs, wie zuvor definiert, in einem Nahrungsmittel- und/oder Lebensmittelerzeugnis.

Gemäß diesem Erfindungsaspekt kann das Nahrungsmittel- und/oder Lebensmittelerzeugnis insbesondere ein Nahrungsergänzungsmittel, ein funktionelles Lebensmittel (*Functional Food*), ein *Novel Food,* ein Lebensmittelzusatzstoff, ein Nahrungszusatz, ein diätetisches Lebensmittel, ein Power-Snack, ein Appetitzügler oder ein Kraft- und/oder Ausdauersport-Supplement sein.

Weitere Ausgestaltungen, Abwandlungen und Variationen der vorliegenden Erfindung sind für den Fachmann beim Lesen der Beschreibung ohne Weiteres erkennbar oder realisierbar, ohne dass er dabei den Rahmen der vorliegenden Erfindung verlässt.

Die vorliegende Erfindung wird anhand der nachfolgenden Ausführungsbeispiele veranschaulicht, welche die vorliegende Erfindung jedoch keinesfalls beschränken sollen, sondern lediglich die beispielhafte und nichtlimitierende Durchführungsweise und Ausgestaltung der vorliegenden Erfindung erläutern sollen.

### AUSFÜHRUNGSBEISPIELE:

### Verwendete Abkürzungen

- 3-BHB-Ethyl = 3-Hydroxybuttersäureethylester (Edukt)
- Glyc. = Glycerin (Edukt)
- 3-BHB-MG = Monoglycerid der 3-Hydroxybuttersäure (erfindungsgemäßes Reaktionsprodukt)
- 3-BHB-DG = Diglycerid der 3-Hydroxybuttersäure (erfindungsgemäßes Reaktionsprodukt)
- 3-BHB-TG = Triglycerid der 3-Hydroxybuttersäure (erfindungsgemäßes Reaktionsprodukt)
- 3-BHB-Dimer-MG = Monoglycerid des Dimers der 3-Hydroxybuttersäure (Reaktionsnebenprodukt)
- 3-BHB-Dimer-DG = Diglycerid des Dimers der 3-Hydroxybuttersäure (Reaktionsnebenprodukt)
- 3-BHB-FS = 3-Hydroxybuttersäure (Reaktionsnebenprodukt)
- 3-BHB-Dimer-FS = Dimer der 3-Hydroxybuttersäure (Reaktionsnebenprodukt)
- 3-BHB-Dimer = dimeres 3-Hydroxybutyrat (Reaktionsnebenprodukt)
- n.d. = nicht bestimmt

### Herstellungsbeispiele

Das erfindungsgemäße Herstellungsverfahren wird anhand der nachfolgenden Ausführungsbeispiele veranschaulicht.

### Herstellung von 3-BHB-Mono-, Di- und Triglycerid-Gemischen

In einem 500-ml-Mehrhalskolben mit Dephlegmator (Partialkondensator) und Destillationsbrücke werden 300 g (R)/(S)-3-Hydroxybuttersäureethylester (d. h. racemischer Ester), 50 g Glycerin und 3,3 g immobilisiertes Enzym (CALB-Lipase auf Polymerträger, abgeleitet von *Candida antarctica,* z. B. Novozym^{®}435 von der Fa. Sigma-Aldrich bzw. Merck oder Lipozym^{®}435 von der Fa. Strem Chemicals, Inc.) vorgelegt. Das Reaktionsgemisch wird unter Rühren bei 70 °C und unter Vakuum (< 500 mbar) für 36 h zur Reaktion gebracht. Danach wird das Enzym abfiltriert und rezykliert und der überschüssige 3-Hydroxybuttersäureethylester unter Vakuum abdestilliert. Der erhaltene Rückstand wird bei Bedarf für 2 bis 4 h im Hochvakuum dampfbehandelt (Dampftemperatur: 160 °C).

Es wird ein Reaktionsprodukt auf Basis eines Gemischs von Mono-, Di- und Triglyceriden der 3-Hydroxybuttersäure mit folgender Zusammensetzung erhalten: 16 % 3-BHB-Monoglycerid, 58,5 % 3-BHB-Diglycerid und 25 % 3-BHB-Triglycerid, welches noch 0,5 % 3-Hydroxybuttersäure als Reaktionsnebenprodukt enthält. Die Charakterisierung erfolgt mittels GC, GC-MS und NMR.

Im Rahmen der Aufreinigung wird die 3-Hydroxybuttersäure entfernt, so dass das Reingemisch erhalten wird. Ein Teil des Gemischs wird einer Auftrennung mittels Chromatographie unterzogen, so dass die verschiedenen Glyceride jeweils als Reinstoffe erhalten werden (d. h. jeweils reines 3-BHB-Monoglycerid, reines 3-BHB-Diglycerid und reines 3-BHB-Triglycerid). Ein anderer Teil des Gemischs wird einer Auftrennung mittels fraktionierter Destillation unterzogen, so dass einerseits eine erste, leichtsiedende Fraktion, welche einen hohen Anteil an Mono- und Diglyceriden der 3-Hydroxybuttersäure und einen geringen Anteil an Triglyceriden der 3-Hydroxybuttersäure umfasst, und andererseits eine zweite, schwersiedende Fraktion, welche einen geringen Anteil an Monoglyceriden der 3-Hydroxybuttersäure und einen hohen Anteil an Di- und Triglyceriden der 3-Hydroxybuttersäure umfasst, erhalten wird.

Die Ergebnisse der Fraktionierung sind in der folgenden Tabelle zusammengefasst (Kurzwegdestillation, 0,01 mbar Druck, Temperatur Vorlagenbehälter: 20 °C, Manteltemperatur 83 °C):

| | Rohgemisch % | Fraktion I Schwersieder % | Fraktion II Leichtsieder % |
|---|---|---|---|
| 3-BHB-Ethyl | 0,6 | 0,05 | 0,6 |
| 3-BHB-FS | 0,7 | 0,5 | 2,7 |
| Glycerin | 1,3 | 0,4 | 10,2 |
| 3-BHB-Dimer | 3,4 | 0,7 | 27,2 |
| 3-BHB-MG | 18 | 15,8 | 45,8 |
| 3-BHB-Dimer-MG | 0,5 | 0,3 | 2,6 |
| 3-BHB-DG | 53,3 | 57,8 | 10 |
| 3-BHB-TG | 15,2 | 16,7 | 0,6 |
| 3-BHB-Dimer-DG | 6,3 | 6,8 | 0,2 |
| Polymere Bestandteile | 0,9 | 1 | - |

### Synthesen mit Enzym als Katalysator

Zur erfindungsgemäßen Synthese von erfindungsgemäßen 3-BHB-Glycerid-Gemischen sind Enzyme grundsätzlich gut geeignet. Sie sind hochselektiv und können bei milden Reaktionsbedingungen eingesetzt werden. Des Weiteren sind Enzyme häufig enantioselektiv. Da es sich bei 3-BHB-FS bzw. deren Estern um bifunktionale Moleküle (Vorhandensein einer OH-Gruppe sowie einer Carboxylgruppe bzw. Estergruppe) handelt, führen herkömmliche chemische Veresterungs- oder Umesterungs-Bedingungen zu vermehrter und unerwünschter Nebenproduktbildung (z. B. Oligo- oder Polymere, Eliminierungsreaktionen etc.). Enzyme haben dagegen das Potential, diese potentielle Nebenproduktbildung durch ihre hohe Selektivität zu umgehen.

Die nachfolgende Abbildung zeigt exemplarisch das Reaktionsschema einer erfindungsgemäßen enzymatischen Veresterung (d. h. Herstellung eines 3-BHB-MG-, 3-BHB-DG- und 3-BHB-TG-Gemisches mittels Enzymkatalyse).

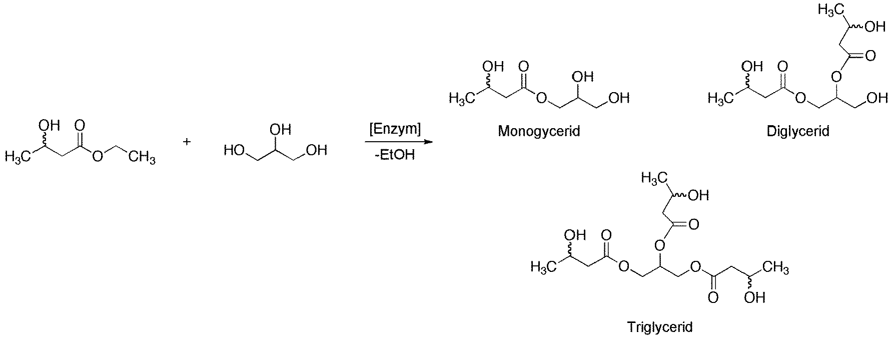

Aber auch bei der Verwendung von Enzymen kann es noch zu geringer Neben- bzw. Folgeproduktbildung kommen. Die nachfolgende Abbildung zeigt typische, bei einer Enzymkatalyse gebildete Nebenprodukte.

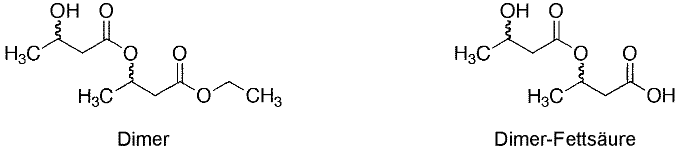

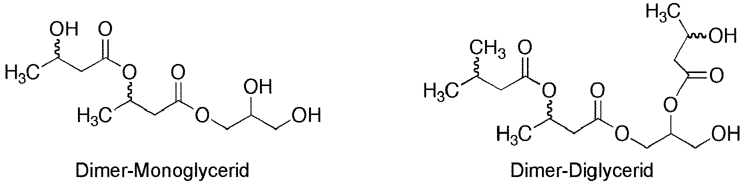

Bei den in der vorstehenden Abbildung gebildeten Nebenprodukten handelt es sich insbesondere um Folgeprodukte. Ohne sich auf eine Theorie festzulegen, lässt sich die Bildung der jeweiligen Dimer-Glyceride möglicherweise dadurch erklären, dass sich die jeweiligen Dimer-Glyceride aus den Dimeren-Ethylestern bilden oder aber die 3-BHB-Monoglyceride und 3-BHB-Diglyceride weiter an der OH-Gruppe umgeestert werden. Die 3-BHB-Dimer-Säure (3-BHB-Dimer-FS) tritt überhaupt nur in Gegenwart von Wasser auf und spielt z. B. für einen Umesterungs-Produktionsprozess mit 3-BHB-Ethyl aufgrund einer möglichen Vakuum-Verfahrensführung nur eine untergeordnete Rolle.

### Testreihen mit Enzymen

Zunächst werden Testreihen mit Enzymen durchgeführt, um geeignete Enzyme bzw. geeignete Enzym-Varianten für die erfindungsgemäße Synthese von 3-BHB-Glycerid-Gemischen zu finden.

Hierzu wird zunächst eine Testreihe zur parallelen Untersuchung von bis zu 4 Ansätzen im Maßstab von 50 g bis 100 g durchgeführt. Die Experimente der Testreihe finden in Erlenmeyer-Kolben statt. Diese werden in einem Wasserbad für 24 h bei der gewünschten Temperatur gehalten. Danach wird das Enzym abfiltriert und gegebenenfalls rezykliert. Der Versuch findet pro Enzym jeweils mit und ohne Deckel statt. Es soll hiermit gezeigt werden, wie groß der Einfluss des sich bildenden Koppelprodukts Ethanol auf die chemische Gleichgewichtslage ist.

Die nachfolgende Tabelle zeigt die Ergebnisse einer Enzym-Testreihe.

**Tabelle: Ergebnisse einer Enzym-Testreihe (50 °C, 24 h, 1 Gew.-% Enzym, 40 Mol-% Überschuss an 3-BHB-Ethylester)**

| | **1** | **2** | **3** | **4** |
|---|---|---|---|---|
| | Novozym^{®} 435 | Novozym^{®} 435 | Lipozym^{®} 435 | Lipozym^{®} 435 |
| | geschlossenes System | offenes System | geschlossenes System | offenes System |
| 3-BHB-FS | 1,59 | 8,95 | 1,61 | 7,58 |
| 3-BHB-Dimer | 8,30 | 5,51 | 8,54 | 6,05 |
| 3-BHB-Dimer-FS | n.d. | 2,15 | n.d. | 1,89 |
| 3-BHB-MG | 59,99 | 22,49 | 59,57 | 25,97 |
| 3-BHB-Dimer-MG | n.d. | 0,84 | n.d. | 0,95 |
| 3-BHB-DG | 27,81 | 48,50 | 27,78 | 48,00 |
| 3-BHB-TG | 1,51 | 4,45 | 1,34 | 4,31 |
| 3-BHB-Dimer-TG | 0,99 | 7,17 | 1,06 | 7,26 |
| Polymere | n.d. | n.d. | n.d. | n.d. |

Die Tabelle zeigt die Zusammensetzung der Reaktionsmischung nach 24 h. Die Tabelle zeigt die prozentuale Zusammensetzung ohne Berücksichtigung der gegebenenfalls noch enthaltenen Edukte. Bei den eingesetzten Enzymen handelt es sich um immobilisierte Enzyme auf polymeren Trägersubstanzen.

Die Ergebnisse der obigen Enzym-Testreihe zeigen, dass Novozym^{®}435 und Lipozym^{®} 435 hohe Ausbeuten an 3-BHB-MG, 3-BHB-DG und 3-BHB-TG liefern.

Die Versuche im offenen System zeigen eine verstärkte Bildung von 3-BHB-FS bzw. 3-BHB-Dimer-FS. Dies liegt daran, dass sich - vor allem bedingt durch das verdampfende Wasserbad - unerwünschtes Wasser im Reaktionsgemisch befindet. Diese Nebenproduktbildung spielt aber z. B. für einen Umesterungs-Produktionsprozess mit 3-BHB-Ethyl eine geringere Rolle, da dieser unter Vakuum durchgeführt werden kann.

Aufgrund der Erkenntnisse der Hydrolyse-Nebenreaktion werden im Weiteren Testreihen-Experimente unter Verwendung von CaCl₂-Trockenröhrchen durchgeführt. Die Ergebnisse dieser Versuche sind in der nachfolgenden Tabelle zusammengefasst.

**Tabelle: Ergebnisse bei Verwendung von Trockenröhrchen (70 °C, 24 h, 1 Gew.-% Enzym)**

| | ohne Enzym | Lipozym^{®} 435 | Novozym^{®} 435 |
|---|---|---|---|
| 3-BHB-Ethyl | 69,9 | 54,9 | 54,7 |
| 3-BHB-FS | 0,0 | 0,6 | 0,6 |
| Glycerin | 26,4 | 8,2 | 8,2 |
| 3-BHB-Dimer | 0,7 | 2,7 | 2,8 |
| 3-BHB-Dimer-FS | 0,0 | 0,0 | 0,0 |
| 3-BHB-MG | 3,2 | 21,5 | 21,6 |
| 3-BHB-Dimer-MG | 0,0 | 0,0 | 0,0 |
| 3-BHB-DG | 0,1 | 11,0 | 11,1 |
| 3-BHB-TG | 0,0 | 0,7 | 0,7 |
| 3-BHB-Dimer-DG | 0,0 | 0,3 | 0,4 |
| Polymere | 0,0 | 0,1 | 0,1 |

Der Vergleich der beiden Enzyme Novozym^{®}435 und Lipozym^{®}435 bei Verwendung eines Trockenröhrchens zeigt, dass die Hydrolyse nur noch in sehr geringem Maße stattfindet. Allerdings unterscheiden sich die Ergebnisse in Bezug auf Ausbeute und Selektivität kaum von vergleichbaren Experimenten im geschlossenen System. Der Druckverlust über das Trockenmittel CaCl₂ ist aber zu hoch, so dass kaum Ethanol entweichen kann. Der Eintrag von Wasser kann jedoch vermieden werden. Die Ergebnisse ohne Enzym zeigen, dass die Reaktion auch autokatalytisch ablaufen kann. Es bilden sich auch hier geringe Mengen an BHB-MG.

Weitergehende Untersuchungen zeigen, dass die Enzyme stereoselektiv sind. Insbesondere kann aus einer weiterführenden Analyse der Dimere abgeleitet werden, dass ein Enantiomer aus der Ausgangsverbindung bevorzugt umgesetzt wird, wahrscheinlich das (R)-Enantiomer, so dass ein deutlich erhöhter Anteil an (R)-3-Hydroxybuttersäure bzw. (R)-3-BHB in den gebildeten Produkten vorhanden ist.

Ein NMR-Spektrum des 3-BHB-Dimers (durch Säulenchromatographie auf > 90 % aufgereinigt) bestätigt die Enantioselektivität des Enzym-Prozesses. Gemäß dem NMR-Spektrum wird ein Diasteroemerenverhältnis von 56 : 44 bestimmt; gemäß GC-Analyse beträgt dieses Verhältnis 59 : 41.

### Testreihen mit Edukt-Überschüssen

Um die Aktivität der beiden ausgewählten Enzyme Novozym^{®}435 sowie Lipozym^{®} 435 bei unterschiedlichen 3-BHB-Ethylester Überschüssen zu testen, werden beide Enzyme bei 50 °C für 24 h äquimolar und mit 100 Mol.-% Überschuss umgeestert. Die nachfolgende Tabelle fasst die Ergebnisse zusammen.

**Tabelle: Ergebnisse der Edukt-Überschuss-Testreihen; 50 °C, 24 h, 1 Gew.-% Enzym (geschlossenes System=g.S.; offenes System=o.S.; Novozym^{®} 435=N. 435; Lipozym^{®} 435=L 435)**

| | **1** | **2** | **3** | **4** | **5** | **6** | **7** | **8** |
|---|---|---|---|---|---|---|---|---|
| | N. 435 | N. 435 | L. 435 | L. 435 | N. 435 | N. 435 | L. 435 | L. 435 |
| | äquim. | äquim | äquim | äquim | 100 % | 100 % | 100 % | 100 % |
| | g.S. | o.S. | g.S. | o.S. | g.S. | o.S. | g.S. | o.S. |
| 3-BHB-FS | 1,25 | 9,01 | 1,28 | 9,42 | 2,23 | 13,83 | 2,09 | 11,66 |
| 3-BHB-Dimer | 4,95 | 2,04 | 5,24 | 2,16 | 13,23 | 10,51 | 13,60 | 11,64 |
| 3-BHB-Dimer-FS | n.d. | 1,80 | n.d. | 2,12 | n.d. | 3,11 | n.d. | 3,22 |
| 3-BHB-MG | 67,59 | 32,63 | 67,26 | 32,34 | 50,91 | 20,03 | 50,94 | 17,69 |
| 3-BHB-Dimer-MG | n.d. | 0,29 | n.d. | 0,38 | 0,71 | 1,34 | 0,72 | 1,80 |
| 3-BHB-DG | 24,64 | 45,49 | 24,60 | 44,26 | 29,75 | 41,66 | 29,61 | 42,25 |
| 3-BHB-TG | 0,89 | 3,07 | 0,88 | 3,05 | 1,78 | 3,61 | 1,69 | 4,32 |
| 3-BHB-Dimer-TG | 0,68 | 5,52 | 0,74 | 6,11 | 1,39 | 5,71 | 1,36 | 7,14 |
| Polymer | n.d. | 0,16 | n.d. | 0,17 | n.d. | 0,19 | n.d. | 0,28 |

Die Ergebnisse zeigen, dass die Versuche im offenen System, d. h. beim Abdestillieren von Ethanol, einen höheren Umsatz zu 3-BHB-DG bzw. 3-BHB-TG zeigen. Des Weiteren zeigt sich auch bei dieser Versuchsreihe die verstärkte Hydrolyse bei offenen Reaktionsgefäßen (d. h. im offenen System). Novozym^{®}435 und Lipozym^{®}435 zeigen hierbei unter gleichen Reaktionsbedingungen ähnliche Ergebnisse. Lipozym^{®} 435 scheint dabei etwas aktiver zu sein als Novozym^{®} 435.

Der Umsatz an Glycerin ist bei den Ansätzen mit 100 Mol-% Überschuss am größten. Der Einfluss auf die Bildung von 3-BHB-Dimeren und der Folgeprodukte (d. h. 3-BHB-Dimer-MG und 3-BHB-Dimer-DG) ist jedoch deutlich zu erkennen. Diese Produkte werden bei 100 Mol-% Überschuss zwei- bis viermal so viel gebildet, während der Anteil an 3-BHB-TG nur rund zweimal so hoch ist.

Zusammenfassend lässt sich feststellen, dass bei den gewählten Reaktionsbedingungen ein Überschuss von 100 Mol-% einen stärkeren Einfluss auf die Nebenproduktbildung im Vergleich zur Hauptproduktbildung hat.

### Testreihen zur Untersuchung von Temperatur- und Substrat-Effekten

Der Einfluss der Temperatur sowie eine erste Abschätzung des Einflusses von Substraten auf die Produktbildung werden ebenfalls in Testreihen-Experimenten untersucht.

Die Reaktionstemperatur wird auf 70 °C eingestellt. Zusätzlich werden bei einigen Ansätzen bzw. Versuchen 1 g einer Schwersieder-Fraktion aus einer KD-Destillation (siehe auch oben) hinzugefügt, um eine erste Abschätzung des Einflusses von Substraten auf die Produktbildung zu untersuchen.

Das mit der Schwersieder-Fraktion gemischte Reaktionsgemisch dieser Ansätze hat nachfolgende Ausgangszusammensetzung:

| | Ausgangszusammensetzung mit Schwersieder-Fraktion |
|---|---|
| 3-BHB-Ethyl | 72,2 |
| 3-BHB-FS | < 0,1 |
| Glycerin | 24,3 |
| 3-BHB-Dimer | 0,7 |
| 3-BHB-Dimer-FS | n.d. |
| 3-BHB-MG | 0,5 |
| 3-BHB-Dimer-MG | n.d. |
| 3-BHB-DG | 1,6 |
| 3-BHB-TG | 0,2 |
| 3-BHB-Dimer-DG | 0,4 |
| Polymere | n.d. |

Die nachfolgende Tabelle zeigt die Ergebnisse der Substrat-Testreihen.

**Tabelle: Ergebnisse der Substrat-Testreihen (70 °C, 24 h, 1 Gew.-% Enzym) (geschlossenes System=g.S.; offenes System=o.S.; Novozym^{®} 435=N. 435; Lipozym^{®} 435=L. 435)**

| | **1** | **2** | **3** | **4** | **5** | **6** | **7** | **8** |
|---|---|---|---|---|---|---|---|---|
| | N. 435 | N. 435 | L. 435 | L. 435 | N. 435 | N. 435 | L. 435 | L. 435 |
| | g.S. | o.S. | g.S. | o.S. | g.S. | o.S. | g.S. | o.S. |
| 3-BHB-FS | 1,65 | - | 1,71 | - | 1,70 | - | 1,71 | - |
| 3-BHB-Dimer | 8,89 | 5,30 | 8,48 | 4,87 | 7,77 | 3,86 | 7,58 | 4,07 |
| 3-BHB-Dimer-FS | n.d. | 3,31 | n.d. | 3,61 | n.d. | 3,78 | n.d. | 3,22 |
| 3-BHB-MG | 55,63 | 16,54 | 55,78 | 13,47 | 55,64 | 14,96 | 56,10 | 14,43 |
| 3-BHB-Dimer-MG | 0,35 | 1,03 | 0,31 | 0,86 | 0,33 | 0,74 | 0,31 | 0,83 |
| 3-BHB-DG | 30,42 | 43,90 | 30,69 | 45,19 | 31,42 | 44,36 | 31,23 | 45,17 |
| 3-BHB-TG | 2,07 | 10,64 | 2,05 | 11,31 | 2,13 | 11,36 | 2,11 | 12,60 |
| 3-BHB-Dimer-DG | 0,98 | 8,20 | 0,99 | 8,32 | 1,01 | 8,41 | 0,97 | 9,20 |
| Polymere | n.d. | 0,91 | n.d. | 0,87 | n.d. | 0,97 | n.d. | 1,15 |
| Summe Nebenprodukte | 11,88 | 18,76 | 11,48 | 18,53 | 10,81 | 17,75 | 10,57 | 18,47 |
| | 40 Mol-% | | | | 40 Mol-% + 1 g Schwersieder-Fraktion | | | |

Festzustellen ist zunächst ein Einfluss der Luftfeuchtigkeit bzw. der daraus folgenden Hydrolyse bei den Versuchen im offenen System. Es zeigen sich die Vorteile des Abdestillierens des entstehenden Ethanols zur Gleichgewichtsverschiebung. Lipozym^{®}435 und Novozym^{®} 435 liefern sehr ähnliche Ergebnisse mit leicht höherer Aktivität beim Lipozym^{®} 435.

Ein Einfluss der zugegebenen Menge an Produkten (1 g einer Schwersieder-Fraktion aus einer KD-Destillation) ist nicht erkennbar. Die Menge addiert sich nahezu vollständig zur gebildeten Produktmenge. Beispielsweise bildet sich 3-BHB-DG bei Novozym^{®}435 (offenes System) mit 35 % ohne Zugabe von Produkten und mit 36,7 % bei Zugabe von Produkten. Diese Erhöhung entspricht genau der zugegebenen Menge an 3-BHB-DG von 1,6% im Rahmen der Messgenauigkeit.

Der Summenparameter der Nebenprodukte beinhaltet alle Dimeren bzw. Dimer-Glyceride (siehe auch obige Darstellung). Freie Fettsäure bzw. deren Dimere sind in synthetischer Hinsicht unbeachtlich, da die Hydrolyse in einem Produktionsprozess eine nur untergeordnete Rolle spielt.

Die Ergebnisse zeigen jedoch auch, dass eine aus einem Produktions-Prozess stammende, abdestillierte Edukt-Fraktion (3-BHB-Ethylester-Fraktion) dem Prozess wieder zugeführt werden kann, ohne einen signifikanten Einfluss auf die Reaktion zu haben.

In weiteren Testreihen unter Variation der Temperatur kann man den Einfluss der Temperatur auf die Produktbildung erkennen. Gegenüber einer Reaktionstemperatur von 50 °C wird beispielsweise etwa 4 % bis 5 % mehr 3-BHB-TG gebildet (bei einem gleichzeitig um etwa 25 % höherem Glycerin-Umsatz).

Auch ist der Einfluss eines offenen Systems gegenüber einem geschlossenen System erkennbar: Im geschlossenen System ist die Bildung von 3-BHB-MG gegenüber 3-BHB-DG bevorzugt, wohingegen sich dies im offenen System in etwa umkehrt. Die Bildung von 3-BHB-TG und den Nebenprodukten bleibt dagegen hiervon in etwa unbeeinflusst.

### Testreihen mit Enzym-Rezyklierungen

In weiteren Testreihen-Experimenten wird die Rezyklierfähigkeit (RecyclingFähigkeit) der verwendeten Enzyme Novozym^{®}435 und Lipozym^{®} 435 untersucht. Hierzu wird das jeweilige Enzym vom vorgehenden Versuch abfiltriert und der Filterrückstand nebst Produktanhaftungen für ein weiteres Experiment eingesetzt.

Es zeigt sich, dass beide Enzyme nach 24 h bei 70 °C rezykliert werden können. Nach dem ersten Rezyklierungs-Zyklus zeigt sich eine kaum verringerte Aktivität, und es werden nahezu identische Zusammensetzungen erhalten. Auffallend ist jedoch, dass beide Enzyme nach Rezyklierung einen geringeren Glycerin-Umsatz aufweisen, aber gleichzeitig weniger Dimere bilden.

Die nachfolgende Tabelle zeigt die erhaltenen Ergebnisse.

**Tabelle: Ergebnisse des Enzym-Recyclings (70 °C, 24 h, 1 Gew.-% Enzym, 40 Mol-% Überschuss BHB-Ethyl-Ester, alle Versuche mit Trockenrohr)**

| | Novozym^{®} 435 frisch | Novozym^{®} 435 rezykliert | Lipozym^{®} 435 frisch | Lipozym^{®} 435 rezykliert |
|---|---|---|---|---|
| 3-BHB-Ethyl | 54,7 | 56,4 | 54,9 | 56,8 |
| 3-BHB-FS | 0,6 | 1,0 | 0,6 | 0,7 |
| Glycerin | 8,2 | 8,8 | 8,2 | 8,8 |
| 3-BHB-Dimer | 2,8 | 1,6 | 2,7 | 1,5 |
| 3-BHB-Dimer-FS | 0,0 | 0,0 | 0,0 | 0,0 |
| 3-BHB-MG | 21,6 | 21,6 | 21,5 | 21,6 |
| 3-BHB-Dimer-MG | 0,0 | 0,0 | 0,0 | 0,0 |
| 3-BHB-DG | 11,1 | 10,0 | 11,0 | 10,0 |
| 3-BHB-TG | 0,7 | 0,5 | 0,7 | 0,5 |
| 3-BHB-Dimer-DG | 0,4 | 0,2 | 0,3 | 0,2 |
| Polymere | 0,1 | 0,0 | 0,1 | 0,0 |

### Testreihen mit Untersuchung der Enzym-Konzentration

Der Einfluss der Enzymkonzentration wird in weiteren Testreihen-Experimenten ebenfalls untersucht. Hierzu werden Ansätze mit 0,1 Gew.-%, 1 Gew.-%, 5 Gew.-% und 10 Gew.-% an Enzym angesetzt. Die nachfolgende Tabelle fasst die Ergebnisse zusammen. Die Versuche wurden je mit Novozym^{®} 435 (offenes System) durchgeführt.

Die Ergebnisse zeigen, dass die optimale Konzentration im Bereich von etwa 1 Gew.-% Enzym bei 70 °C liegt. Hier wird der höchste Glycerinumsatz erzielt. Bei höheren Enzym-Konzentrationen sinkt der Glycerinumsatz wieder ab. Des Weiteren führen Enzymmengen ≥ 5 % zu einer verstärkten Bildung von Dimer-DG und höheren Dimer-Verbindungen. Bei allen Versuchen ist der erhöhte Anteil an BHB-FS und BHB-Dimer-FS auf die offene Versuchsweise (d. h. im offenen System) im Wasserbad zurückzuführen.

**Tabelle: Ergebnisse der Enzym-Konzentration-Testreihen (70 °C, 24 h, Novozym^{®} 435, 40 Mol-% Überschuss 3-BHB-Ethylester)**

| | 0,1 Gew.-% | 1,0 Gew.-% | 5,0 Gew.-% | 10,0 Gew.-% |
|---|---|---|---|---|
| 3-BHB-FS | 5,12 | 13,50 | 11,86 | 11,11 |
| 3-BHB-Dimer | 1,90 | 2,53 | 2,81 | 3,39 |
| 3-BHB-Dimer-FS | 0,30 | 3,41 | 6,24 | 6,39 |
| 3-BHB-MG | 42,73 | 18,05 | 18,18 | 18,35 |
| 3-BHB-Dimer-MG | 0,00 | 0,37 | 0,68 | 1,47 |
| 3-BHB-DG | 46,14 | 46,30 | 34,06 | 32,56 |
| 3-BHB-TG | 2,40 | 8,54 | 9,78 | 9,51 |
| 3-BHB-Dimer-DG | 1,42 | 6,79 | 11,89 | 12,48 |
| Polymere | - | 0,51 | 1,60 | 1,63 |
| Unbekannte | - | - | 2,89 | 3,12 |
| Summe Nebenprodukte | 8,73 | 27,10 | 35,09 | 36,46 |

### Synthesen mit basischem Metallkatalysator (nicht erfindungsgemäß)

Eine weitere Möglichkeit zur Synthese von 3-BHB-Glycerid-Gemischen besteht grundsätzlich in der chemischen Synthese unter Verwendung von Metallkatalysatoren, Säuren oder Basen. Hierzu wird ausgehend von der freien Fettsäure unter Wasserabspaltung eine Veresterung durchgeführt oder bei Verwendung des z. B. 3-BHB-Ethylesters unter Ethanolabspaltung eine Umesterung durchgeführt (und zwar jeweils in Gegenwart des Katalysators). Durch geeignete Reaktionsführung können im Rahmen der vorliegenden Erfindung die im Stand der Technik üblicherweise auftretenden Nebenreaktionen, insbesondere Oligo- und Polymerisationsreaktionen, effizient minimiert bzw. vermieden werden.

Das nachfolgende Reaktionsschema zeigt eine mögliche chemische Synthese von 3-BHB-Glyceridgemischen durch Umesterung mit basischem Katalysator.

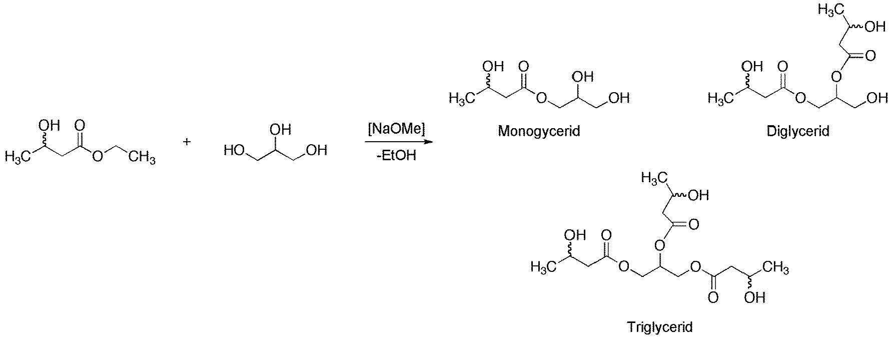

Die nachfolgende Tabelle zeigt die Ergebnisse einer Umsetzung von 3-BHB-Ethylester mit Natriummethylat (NaOMe) und Glycerin.

**Tabelle: Ergebnisse der Umsetzung von 3-BHB-Ethyl-Ester mit Natriummethylat (NaOMe) und Glycerin (100 °C, 12 h, 1 Gew.-% NaOMe, 40 Mol-% Überschuss BHB-Ethylester, Vakuum)**

| | |
|---|---|
| 3-BHB-Methyl | 2,7 |
| 3-BHB-Ethyl (Edukt) | 49,8 |
| 3-BHB-FS | 1,9 |
| Glycerin | 13,4 |
| 3-BHB-Dimer | 4,2 |
| 3-BHB-Dimer-FS | 0,0 |
| 3-BHB-MG | 20,6 |
| 3-BHB-Dimer-MG | 0,2 |
| 3-BHB-DG | 6,8 |
| 3-BHB-TG | 0,3 |
| 3-BHB-Dimer-DG | 0,2 |
| Polymere | 0,0 |

Es zeigt sich, dass Polymerisationen nicht sattfinden. Es bilden sich Mono-, Di- und Triglyceride und geringe Mengen an Dimeren. Eine weitergehende Zugabe von NaOMe und/oder die Anwendung von geringerem Vakuum können zwar den Reaktionsfortschritt nicht weiter erhöhen, da offenbar ein Gleichgewichtszustand oder eine kinetische Limitierung erreicht zu sein scheint, lassen aber selektiv nur den Anteil an 3-BHB-Methylester ansteigen, so dass davon auszugehen ist, dass der Katalysator weiterhin aktiv ist und sich auf diese Weise die Reaktion steuern lässt.

### Physiologische Anwendungsversuche: in-vitro-Verdauversuche

### Verdauversuche (Spalt- bzw. Spaltungsversuche) von erfindungsgemäßen 3-BHB-Glycerid-Gemischen

Mittels Spaltungsversuchen wird gezeigt, dass erfindungsgemäß hergestellte 3-BHB-Ester bzw. deren Gemische, einschließlich der Reaktionsnebenprodukte wie Dimeren etc., im menschlichen gastrointestinalen Trakt gespalten werden können. Als Ausgangsgemisch wird eine nicht weiter aufgereinigte, nach dem erfindungsgemäßen Verfahren erhaltene Roh-Schwersiederfraktion aus der fraktionierten Destillation eingesetzt, welche ein erfindungsgemäß erhaltenes ternäres Gemisch von 3-BHB-MG, 3-BHB-DG und 3-BHB-TG enthält.

Für die Spaltungsversuche unter köpernahen Bedingungen werden zwei Medien untersucht:
- FaSSGF, welches den Magen simuliert
- FaSSIF, welches den Darmtrakt simuliert

Beide Medien stammen von der Firma Biorelevant^{®}, Ltd., Großbritannien. Zusätzlich wird in einigen Experimenten Schweine-Pankrease zugesetzt (Panzytrat°40.000, Fa. Allergan).

Die Ergebnisse der Experimente sind in der nachfolgenden Tabelle zusammengefasst (chromatographisch bestimmt, Prozentangabe jeweils bestimmt als % Fläche im Chromatogramm).

**Tabelle: Ergebnisse der Spaltungsversuche in einem FaSSGF- bzw. FaSSIF-Medium mit Panzytrat^{®} (m.P.) und ohne Panzytrat^{®} (o.P.) (35 °C, 24 h)**

| | vor Spaltung | FaSSGF (Magen, pH = 1,6) | | FaSSIF (Darm, pH = 6,5) | | |
|---|---|---|---|---|---|---|
| | | o.P. 24 h, 35 °C | m.P. 24 h, 35 °C | 0 h, Start | o.P. 24 h, 35 °C | m.P. 24 h, 35 °C |
| | % Fläche | % Fläche | % Fläche | % Fläche | % Fläche | % Fläche |
| 3-BHB-Ethyl | 0,31 | 0,07 | 0,11 | 0,16 | 0,18 | 0,20 |
| 3-BHB-FS | 0,43 | 26,21 | 18,85 | 0,04 | 0,02 | 2,60 |
| Glycerin | 0,38 | 0,81 | 0,51 | 0,07 | 0,05 | 0,07 |
| 3-BHB-Dimer | 0,71 | 0,35 | 0,45 | 0,70 | 0,72 | 0,72 |
| 3-BHB-Dimer-FS | n.d. | 3,25 | 2,51 | n.d. | n.d. | 0,17 |
| 3-BHB-MG | 18,28 | 21,09 | 18,41 | 13,52 | 12,26 | 14,34 |
| 3-BHB-Dimer-MG | 0,39 | 0,16 | 0,22 | 0,42 | 0,44 | n.d. |
| 3-BHB-DG | 55,85 | 37,01 | 44,09 | 60,17 | 60,93 | 61,38 |
| 3-BHB-TG | 16,08 | 7,55 | 10,12 | 16,40 | 16,79 | 12,83 |
| 3-BHB-Dimer-DG | 6,62 | 3,14 | 4,22 | 7,56 | 7,61 | 7,02 |
| Polymere | 0,95 | 0,36 | 0,52 | 0,97 | 1,01 | 0,69 |

Es zeigt sich, dass die Probe unter FaSSGF-Bedingungen hydrolysiert. Dies liegt hauptsächlich am niedrigen pH-Wert (pH = 1,6).

Bei FaSSIF-Bedingungen findet eine geringe Umsetzung bzw. Spaltung (2,6 % 3-BHB-FS) unter Verwendung von Panzytrat^{®} statt.

Bei allen Experimenten ist zu erkennen, dass sich die Kaskade (3-BHB-TG wird zu 3-BHB-DG, 3-BHB-DG wird zu 3-BHB-MG, 3-BHB-MG wird zu freier Säure und Glycerin) fortsetzt. Des Weiteren bilden sich auch Dimer-Säuren (3-BHB-Dimer-FS). Daraus lässt sich erkennen, dass auch die Dimer-Glyceride abgebaut werden. Dies lässt sich folglich für einen etwaigen pharmazeutischen bzw. pharmakologischen Retardierungs-Effekt nutzen.

### Weitere Verdauversuche (Spaltungsversuche) von erfindungsgemäßen 3-BHB-Glycerid-Gemischen

### 1. Spaltungsversuche mit Pankreatin

2 g eines wie zuvor beschrieben hergestellten Glyceridgemischs auf Basis von Mono-, Di- und Triglyceriden der 3-Hydroxybuttersäure werden in 50 g Wasser gelöst und mit 0,5 g (1 Gew.-%) Pankreatin versetzt.

Das Pankreatin wird in Form des kommerziell verfügbaren Produkts Panzytrat^{®} 40.000 von der Fa. Allergan eingesetzt.

Das Ganze wird auf einer Heizplatte bei 50 °C gerührt; der Reaktionsverlauf wird mittels kontinuierlicher Erfassung der Säurezahl über die Zeit ermittelt und verfolgt.

Die Säurezahl steigt über einen Zeitraum von 1.250 min von ursprünglich 0,200 mg KOH/g auf über 2,200 mg KOH/g an (Spaltung der 3-BHB-Glyceride zu der freien Säure).

Der Umsatz/Zeit-Verlauf der wässrigen Spaltung des erfindungsgemäßen Glyceridgemischs mittels Pankreatin, einschließlich Zunahme der Säurezahl über die Zeit, belegt die gewünschte Zersetzung des Eduktgemischs (Glyceridgemischs) zur freien Säure. Dies wird nachfolgend durch entsprechende Analytik bestätigt.

Der Versuch belegt, dass das erfindungsgemäße Glyceridgemisch aus Mono-, Di- und Triglyceriden von 3-Hydroxybuttersäure ein geeigneter physiologischer Präkursor für 3-Hydroxybuttersäure für die entsprechenden Ketokörpertherapien darstellt.

Der Versuch wird anhand der einzelnen Glyceride wiederholt und verifiziert. Es werden vergleichbare Ergebnisse erhalten, d. h. sowohl das Monoglycerid als auch das Diglycerid sowie auch das Triglycerid der 3-Hydroxybuttersäure werden durch Pankreatin jeweils zu der freien 3-Hydroxybuttersäure gespalten.

### 2. Spaltung mit Magenmedium: FaSSGF-Medium

Ein sogenanntes FaSSGF-Medium wird nach Herstellerangaben aus der kommerziell verfügbaren entsprechenden Zusammensetzung zubereitet (erhältlich von der Fa. Biorelevant, Ltd., Großbritannien).

Die resultierende Probe wird in zwei gleichgroße Chargen aufgeteilt. Jeweils 10 Gew.-% des erfindungsgemäßen ternären Glyceridgemischs auf Basis von Mono-, Di- und Triglyceriden der 3-Hydroxybuttersäure, wie zuvor beschrieben, werden im FaSSGF-Medium gelöst und für 24 Stunden bei 35 °C im Wasserbad belassen. Einer der beiden Chargen ist zuvor zusätzlich 1 Gew.-% Pankreatin (Panzytrat^{®} 40.000) zugesetzt worden.

Zu Zwecken der Analyse werden die Probenlösungen zur Adsorption auf einer CHROMABOND^{®}-Xtr-Säule gegeben und > 5 min einwirken gelassen und dann mit 6 ml DCM (Dichlormethan)/Isopropanol (4 : 1) eluiert.

Die Ergebnisse des Spaltungsversuchs sowohl mit als auch ohne Pankreatin im FaSSGF-Medium zeigen eine starke Zunahme an freier 3-Hydroxybuttersäure und eine deutliche Abnahme der Di- und Triglyceriden der 3-Hydroxybuttersäure im FaSSGF-Medium sowohl mit als auch ohne Pankreatin. Die weiterführende Analytik belegt eine wunschgemäße Aufspaltung bzw. Zersetzung der Probe durch das Medium (pH-Wert des Mediums: 1,6).

Die nachfolgende Tabelle zeigt die Ergebnisse des Spaltungsversuchs mit und ohne Panzytrat^{®} 40.000 im FaSSGF-Medium.

**Tabelle: Ergebnisse der Spaltungsversuche mit und ohne Panzytrat^{®} 40.000 in einem FaSSGF-Medium.**

| | % Fläche | % Fläche | % Fläche |
|---|---|---|---|
| 3-BHB-Ethyl | 0,31 | 0,07 | 0,11 |
| 3-BHB-FS | 0,43 | 26,21 | 18,85 |
| Glycerin | 0,38 | 0,81 | 0,51 |
| 3-BHB-Dimer | 0,71 | 0,35 | 0,45 |
| 3-BHB-Dimer-FS | n.d. | 3,25 | 2,51 |
| 3-BHB-MG | 18,28 | 21,09 | 18,41 |
| 3-BHB-Dimer-MG | 0,39 | 0,16 | 0,22 |
| 3-BHB-DG | 55,85 | 37,01 | 44,09 |
| 3-BHB-TG | 16,08 | 7,55 | 10,12 |
| 3-BHB-Dimer-DG | 6,62 | 3,14 | 4,22 |
| Polymere | 0,95 | 0,36 | 0,52 |
| | vor Spaltung | ohne Panzytrat^{®} | mit Panzytrat^{®} |

### 3. Spaltung mit Darmmedium: FaSSIF-Medium

Das FaSSIF-Medium wird nach Herstellerangaben zubereitet (Fa. Biorelevant, Ltd., Großbritannien).

Die erhaltene Probe wird in zwei gleichgroße Ansätze aufgeteilt. Jeweils 10 Gew.-% des zuvor genannten Glyceridgemischs auf Basis einer Mischung von Mono-, Di- und Triglyceriden der 3-Hydroxybuttersäuren werden im FaSSIF-Medium gelöst und für 24 Stunden bei 35 °C im Wasserbad belassen. Einem der beiden Ansätze ist zuvor zusätzlich 1 Gew.-% Pankreatin (Panzytrat^{®} 40.000) zugesetzt worden.

Zu Zwecken der Analyse werden jeweils 0,5 ml Probenlösung zur Adsorption auf eine CHROMABOND^{®}-Xtr-Säule gegeben und 5 min einwirken gelassen und dann mit 6 ml DCM/Isopropanol (4 : 1) eluiert.

Die Ergebnisse des Spaltungsversuchs mit und ohne Pankreatin im FaSSIF-Medium zeigen eine leichte Zunahme an freier 3-Hydroxybuttersäure und eine entsprechend leichte Abnahme im Gehalt an Triglyceriden der 3-Hydroxybuttersäure des Ausgangsgemischs, was einen enzymatischen Abbau bzw. Zersetzung der Probe belegt, was durch weiterführende Analytik bestätigt wird.

Der pH-Wert des FaSSIF-Mediums liegt bei 6,5. Das FaSSIF-Medium selbst scheint eine Spaltung nicht zu begünstigen.

Die nachfolgende Tabelle zeigt die Ergebnisse des Spaltungsversuchs im FaSSIF-Medium.

**Tabelle: Ergebnisse des Spaltungsversuchs mit und ohne Panzytrat^{®} 40.000 in einem FaSSIF-Medium.**

| | vor Spaltung 0 h | ohne Panzytrat^{®} 24 h, 35 °C | mit Panzytrat^{®} 24 h, 35 °C |
|---|---|---|---|
| 3-BHB-Ethyl | 0,16 | 0,18 | 0,20 |
| 3-BHB-FS | 0,04 | 0,02 | 2,60 |
| Glycerin | 0,07 | 0,05 | 0,07 |
| 3-BHB-Dimer | 0,70 | 0,72 | 0,72 |
| 3-BHB-Dimer-FS | 0,00 | 0,00 | 0,17 |
| 3-BHB-MG | 13,52 | 12,26 | 14,34 |
| 3-BHB-Dimer-MG | 0,42 | 0,44 | 0,00 |
| 3-BHB-DG | 60,17 | 60,93 | 61,38 |
| 3-BHB-TG | 16,40 | 16,79 | 12,83 |
| 3-BHB-Dimer-DG | 7,56 | 7,61 | 7,02 |
| Polymere | 0,97 | 1,01 | 0,69 |

### 4. Weitere Spaltung mit Darmmedium: FaSSIF-Medium

Das FaSSIF-Medium wird entsprechend dem vorangehenden Versuch nach Herstellerangaben zubereitet.

Die resultierende Probe wird in zwei gleichgroße Chargen aufgeteilt. Jeweils 10 Gew.-% des erfindungsgemäßen ternären Glyceridgemischs von Mono-, Di- und Triglyceriden der 3-Hydroxybuttersäuren werden im FaSSIF-Medium gelöst und für 24 Stunden bei 35 °C im Wasserbad belassen. Einer Charge ist zuvor 1 Gew.-% einer porcinen Pankreas-Lipase Typ II (PPL Typ II) zugesetzt worden.

Zu Analysezwecken werden 0,5 ml Probenlösung zur Adsorption auf eine CHROMABOND^{®}-Xtr-Säule gegeben und mehr als 5 min einwirken gelassen und dann mit 6 ml DCM/Isopropanol (4 : 1) eluiert.

Die Ergebnisse des Spaltungsversuchs mit porciner Pankreas-Lipase im FaSSIF-Medium zeigen eine leichte Zunahme an freier 3-Hydroxybuttersäure und eine entsprechende leichte Abnahme im Gehalt an Triglycerid der 3-Hydroxybuttersäure, was einen enzymatischen Abbau bzw. Zersetzung der Probe belegt, was durch weiterführende Analytik bestätigt wird.

Der pH-Wert des FaSSIF-Mediums liegt bei 6,5. Das FaSSIF-Medium selbst scheint eine Spaltung nicht zu begünstigen.

Die nachfolgende Tabelle zeigt die Ergebnisse des Spaltungsversuchs mit und ohne Pankreas-Lipase Typ II im FaSSIF-Medium.

**Tabelle: Ergebnisse der Spaltungsversuche mit und ohne porciner Pankreas-Lipase in einem FaSSIF-Medium**

| | vor Spaltung 0 h | ohne Enzym 24 h, 35 °C | mit Enzym, 24 h, 35 °C |
|---|---|---|---|
| 3-BHB-Ethyl | 0,16 | 0,18 | 0,12 |
| 3-BHB-FS | 0,04 | 0,02 | 1,79 |
| Glycerin | 0,07 | 0,05 | 0,08 |
| 3-BHB-Dimer | 0,70 | 0,72 | 0,71 |
| 3-BHB-Dimer-FS | 0,00 | 0,00 | 0,17 |
| 3-BHB-MG | 13,52 | 12,26 | 15,98 |
| 3-BHB-Dimer-MG | 0,42 | 0,44 | 0,16 |
| 3-BHB-DG | 60,17 | 60,93 | 59,84 |
| 3-BHB-TG | 16,40 | 16,79 | 13,38 |
| 3-BHB-Dimer-DG | 7,56 | 7,61 | 7,07 |
| Polymere | 0,97 | 1,01 | 0,70 |

Die zuvor geschilderten Spaltungsversuche belegen, dass Mono-, Di- und/oder Triglyceride der 3-Hydroxybuttersäure effiziente Präkursoren bzw. Metabolite der freien Hydroxybuttersäure bzw. deren Salzen darstellen, insbesondere im Hinblick auf ihre beabsichtigte Wirkung, welche in physiologisch verträglicher bzw. physiologisch kompatibler Form vorliegen.

## Patentansprüche

1. Verfahren zur Herstellung von Glyceriden der 3-Hydroxybuttersäure (beta-Hydroxybuttersäure, BHB bzw. 3-BHB),
wobei mindestens eine Verbindung der allgemeinen Formel (I)
CH₃-CH(OH)-CH₂-C(O)OR¹ (I)
wobei in der allgemeinen Formel (I) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt,
mit Glycerin (1,2,3-Propantriol) der Formel (II)
CH₂(OH) - CH(OH) - CH₂(OH) (II)
umgesetzt wird,
wobei die Umsetzung in Gegenwart eines Enzyms als Katalysator durchgeführt wird und
wobei die Umsetzung in Abwesenheit von Lösemitteln und/oder ohne jedwedes Lösemittel durchgeführt wird,
so dass als Reaktionsprodukt ein Gemisch von Mono-, Di- und Triglyceriden der 3-Hydroxybuttersäure der allgemeinen Formel (III)
CH₂(OR²) - CH(OR³) - CH₂(OR⁴) (III)
erhalten wird, wobei in der allgemeinen Formel (III) die Reste R², R³ und R⁴, jeweils unabhängig voneinander, Wasserstoff oder einen Rest der Formel CH₃-CH(OH)-CH₂-C(O)-darstellen, jedoch mit der Maßgabe, dass mindestens ein Rest R², R³ und R⁴ keinen Wasserstoff darstellt,
wobei das Gemisch Monoglyceride der 3-Hydroxybuttersäure (3-BHB-MG), Diglyceride der 3-Hydroxybuttersäure (3-BHB-DG) und Triglyceride der 3-Hydroxybuttersäure (3-BHB-TG) in einem gewichtsbezogenen Verhältnis von 3-BHB-MG/3-BHB-DG/3-BHB-TG im Bereich von 10-80 / 10-70 / 0,1-20 umfasst und
wobei bei der Umsetzung gleichzeitig die Verbindung gemäß der allgemeinen Formel (IV)
R¹-OH (IV)
gebildet wird, wobei in der allgemeinen Formel (IV) der Rest R¹ Wasserstoff oder ein C₁-C₄-Alkyl, insbesondere ein C₁-C₄-Alkyl, bevorzugt Methyl oder Ethyl, besonders bevorzugt Ethyl, darstellt, wobei die Verbindung gemäß der allgemeinen Formel (IV) der Umsetzung kontinuierlich entzogen wird.

2. Verfahren nach Anspruch 1,
wobei Verbindung der allgemeinen Formel (I) in racemischer Form oder in Form des (R)-Enantiomers eingesetzt wird; und/oder
wobei in der allgemeinen Formel (I) der Rest R¹ Ethyl darstellt; und/oder
wobei als Verbindung der allgemeinen Formel (I) 3-Hydroxybuttersäureethylester (Ethyl-3-hydroxybutyrat) der Formel CH₃-CH(OH)-CH₂-C(O)OC₂H₅ eingesetzt wird.

3. Verfahren nach Anspruch 1 oder Anspruch 2,
wobei der Katalysators nach Umsetzung rezykliert wird; und/oder
wobei das Enzym ausgewählt wird aus Synthetasen (Ligasen), Katalasen, Esterasen, Lipasen und deren Kombinationen; und/oder
wobei das Enzym sich ableitet von *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* und *Pseudomonas* sp. sowie deren Kombinationen, vorzugsweise von *Candida antarctica, Mucor miehei (Rhizomucor miehei)* und *Thermomyces lanuginosus;* und/oder
wobei das Enzym in immobilisierter Form, insbesondere immobilisiert auf einem Träger, vorzugsweise auf einem polymeren Träger, bevorzugt auf einem polymeren organischen Träger, besonders bevorzugt mit hydrophoben Eigenschaften, ganz besonders bevorzugt auf einem poly(meth)acrylharzbasierten Träger, eingesetzt wird; und/oder
wobei das Enzym nach der Umsetzung rezykliert wird; und/oder
wobei die Umsetzung in Gegenwart eines Enzyms als Katalysator bei Temperaturen im Bereich von 10 °C bis 80 °C, insbesondere im Bereich von 20 °C bis 80 °C, vorzugsweise im Bereich von 25 °C bis 75 °C, besonders bevorzugt im Bereich von 45 °C bis 75 °C, ganz besonders bevorzugt im Bereich von 50 °C bis 70 °C, durchgeführt wird; und/oder
wobei das Enzym in Mengen, bezogen auf die Gesamtmenge der Ausgangsverbindungen der Formeln (I) und (II), im Bereich von 0,001 Gew.-% bis 20 Gew.-%, insbesondere im Bereich von 0,01 Gew.-% bis 15 Gew.-%, vorzugsweise im Bereich von 0,1 Gew.-% bis 15 Gew.-%, bevorzugt im Bereich von 0,5 Gew.-% bis 10 Gew.-%, eingesetzt wird; und/oder
wobei die Umsetzung in Gegenwart eines Enzyms als Katalysator bei einem Druck im Bereich von 0,0001 bar bis 10 bar, insbesondere im Bereich von 0,001 bar bis 5 bar, vorzugsweise im Bereich von 0,01 bar bis 2 bar, besonders bevorzugt im Bereich von 0,05 bar bis 1 bar, ganz besonders bei etwa 1 bar, durchgeführt wird.

4. Verfahren nach einem der vorangehenden Ansprüche,
wobei die Verbindung der allgemeinen Formel (I), bezogen auf die Hydroxylgruppen des Glycerins der Formel (II), in molaren Mengen in einem Bereich von äquimolarer Menge bis zu einem molaren Überschuss von 200 Mol-%, insbesondere in einem Bereich von äquimolarer Menge bis zu einem molaren Überschuss von 150 Mol-%, vorzugsweise in einem Bereich von äquimolarer Menge bis zu einem molaren Überschuss von 100 Mol-%, eingesetzt wird; und/oder
wobei die Verbindung der allgemeinen Formel (I) und das Glycerin der Formel (II) in einem Molverhältnis von Verbindung der allgemeinen Formel (I) / Glycerin der Formel (II) in einem Bereich von 1 :1 bis 10 : 1, insbesondere in einem Bereich von 2 : 1 bis 8 : 1, vorzugsweise in einem Bereich von 3 : 1 bis 6 : 1, eingesetzt wird.

5. Verfahren nach einem der vorangehenden Ansprüche,
wobei das erhaltene Reaktionsprodukt nach erfolgter Umsetzung fraktioniert wird, insbesondere destillativ fraktioniert wird;
insbesondere wobei das Reaktionsprodukt mindestens aufgetrennt wird in eine erste, insbesondere leichtsiedende Fraktion, welche einen hohen Anteil an Mono- und Diglyceriden der 3-Hydroxybuttersäure und einen geringen Anteil an Triglyceriden der 3-Hydroxybuttersäure umfasst, und eine zweite, insbesondere schwersiedende Fraktion, welche einen geringen Anteil an Monoglyceriden der 3-Hydroxybuttersäure und einen hohen Anteil an Di- und Triglyceriden der 3-Hydroxybuttersäure umfasst;
insbesondere wobei die erste, insbesondere leichtsiedende Fraktion ein gewichtsbezogenes Verhältnis von 3-BHB-MG/3-BHB-DG/3-BHB-TG im Bereich von 70-95 / 5-30 / 0,01-2, insbesondere im Bereich von 75-85 / 10-25 / 0-1, umfasst und/oder insbesondere wobei die zweite, insbesondere schwersiedende Fraktion ein gewichtsbezogenes Verhältnis von 3-BHB-MG/3-BHB-DG/3-BHB-TG im Bereich von 5-30 / 20-80 / 5-40, insbesondere im Bereich von 5-20 / 40-75 / 10-30, umfasst.

6. Reaktionsprodukt, erhältlich gemäß dem Verfahren nach einem der Ansprüche 1 bis 5.

7. Gemisch von Mono-, Di- und Triglyceriden der 3-Hydroxybuttersäure der allgemeinen Formel (III)
CH₂(OR²) - CH(OR³) - CH₂(OR⁴) (III)
wobei in der allgemeinen Formel (III) die Reste R², R³ und R⁴, jeweils unabhängig voneinander, Wasserstoff oder einen Rest der Formel CH₃-CH(OH)-CH₂-C(O)-darstellen, jedoch mit der Maßgabe, dass mindestens ein Rest R², R³ und R⁴ keinen Wasserstoff darstellt,
wobei das Gemisch Monoglyceride der 3-Hydroxybuttersäure (3-BHB-MG), Diglyceride der 3-Hydroxybuttersäure (3-BHB-DG) und Triglyceride der 3-Hydroxybuttersäure (3-BHB-TG) in einem gewichtsbezogenen Verhältnis von 3-BHB-MG/3-BHB-DG/3-BHB-TG im Bereich von 10-80 / 10-70 / 0,1-20 umfasst.

8. Gemisch nach Anspruch 7,
wobei das Gemisch Monoglyceride der 3-Hydroxybuttersäure (3-BHB-MG), Diglyceride der 3-Hydroxybuttersäure (3-BHB-DG) und Triglyceride der 3-Hydroxybuttersäure (3-BHB-TG) in einem gewichtsbezogenen Verhältnis von 3-BHB-MG/3-BHB-DG/ 3-BHB-TG im Bereich von 20-70 / 20-60 / 0,5-15, umfasst.

9. Gemisch nach einem der Anspruch 7 oder Anspruch 8,
wobei das Gemisch ein Gemisch von Mono-, Di- und Triglyceriden der 3-Hydroxybuttersäure umfasst, wobei das Gemisch von Mono-, Di- und/oder Triglyceriden der 3-Hydroxybuttersäure die Mono-, Di- und/oder Triglyceride der 3-Hydroxybuttersäure in einem gewichtsbezogenen Verhältnis von 3-BHB-MG/3-BHB-DG/3-BHB-TG im Bereich von 70-95 / 5-30 / 0,01-2, insbesondere im Bereich von 75-85 / 10-25 / 0-1, umfasst; oder
wobei das Gemisch ein Gemisch von Mono-, Di- und Triglyceriden der 3-Hydroxybuttersäure umfasst, wobei das Gemisch von Mono-, Di- und/oder Triglyceriden der 3-Hydroxybuttersäure die Mono-, Di- und/oder Triglyceride der 3-Hydroxybuttersäure in einem gewichtsbezogenen Verhältnis von 3-BHB-MG/3-BHB-DG/3-BHB-TG im Bereich von 5-30 / 20-80 / 5-40, insbesondere im Bereich von 5-20 / 40-75 / 10-30, umfasst.

10. Pharmazeutische Zusammensetzung, insbesondere Arzneimittel oder Medikament, umfassend ein Reaktionsprodukt gemäß Anspruch 6 und/oder ein Gemisch gemäß einem der Ansprüche 7 bis 9.

11. Pharmazeutische Zusammensetzung nach Anspruch 10 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glukosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

12. Reaktionsprodukt gemäß Anspruch 6 und/oder Gemisch gemäß einem der Ansprüche 7 bis 9 zur Verwendung bei der prophylaktischen und/oder therapeutischen Behandlung von Erkrankungen des menschlichen oder tierischen Körpers, insbesondere Erkrankungen im Zusammenhang mit einer Störung des Energiestoffwechsels, insbesondere Ketokörperstoffwechsels, wie insbesondere Schädel-Hirn-Trauma, Schlaganfall, Hypoxien, kardiovaskuläre Erkrankungen wie Myokardinfarkt, Refeeding-Syndrom, Anorexien, Epilepsie, neurodegenerative Erkrankungen wie Demenz, Morbus Alzheimer, Morbus Parkinson, Multiple Sklerose und Amyotrophe Lateralsklerose, Fettstoffwechselerkrankungen wie Glukosetransporter-Defekt (GLUT1-Defekt), VL-FAOD und Mitochondriopathien wie mitochondrialer Thiolase-Defekt, Chorea Huntington, Krebserkrankungen wie T-Zell-Lymphome, Astrozytome und Glioblastome, HIV, rheumatische Erkrankungen wie rheumatoide Arthritis und Arthritis urica, Erkrankungen des Gastrointestinaltrakts wie chronisch entzündliche Darmerkrankungen, insbesondere Colitis ulcerosa und Morbus Crohn, lyosomale Speicherkrankheiten wie Sphingolipidosen, insbesondere Niemann-Pick-Erkrankung, Diabetes mellitus und Auswirkungen oder Nebenwirkungen von Chemotherapien.

13. Reaktionsprodukt gemäß Anspruch 6 und/oder ein Gemisch gemäß einem der Ansprüche 7 bis 9 zur Anwendung bei katabolen Stoffwechsellagen, insbesondere Hunger, Diäten oder kohlenhydratarmer Ernährung.

14. Reaktionsprodukt gemäß Anspruch 6 und/oder ein Gemischs gemäß einem der Ansprüche 7 bis 9 zur Verwendung bei der prophylaktischen und/oder therapeutischen Behandlung von katabolen Stoffwechsellagen.

15. Nahrungsmittel- und/oder Lebensmittelerzeugnis, umfassend ein Reaktionsprodukt gemäß Anspruch 6 und/oder ein Gemisch gemäß einem der Ansprüche 7 bis 9.

16. Verwendung eines Reaktionsprodukts gemäß Anspruch 6 und/oder eines Gemischs gemäß einem der Ansprüche 7 bis 9 in einem Nahrungsmittel- und/oder Lebensmittelerzeugnis.

## Claims

1. Process for the preparation of glycerides of 3-hydroxybutyric acid (beta-hydroxybutyric acid, BHB or 3-BHB),
wherein at least one compound of the general formula (I) CH₃ - CH(OH) - CH₂ - C(O)OR¹ (I)
wherein, in the general formula (I), the radical R¹ is hydrogen or a C₁-C₄-alkyl, in particular a C₁-C₄-alkyl, preferably methyl or ethyl, particularly preferably ethyl,
is reacted with glycerol (1,2,3-propanetriol) of the formula (II)
CH₂(OH) - CH(OH) - CH₂(OH) (II)
wherein the reaction is carried out in the presence of an enzyme as catalyst, and
wherein the reaction is carried out in the absence of solvent and/or without any solvent,
so that the reaction product is a mixture of mono-, di- and triglycerides of 3-hydroxybutyric acid of the general formula (III)
CH₂(OR²) - CH(OR³) - CH₂(OR⁴) (III)
in which general formula (III) the radicals R², R³ and R⁴, in each case independently of one another, represent hydrogen or a radical of the formula CH₃ - CH(OH) - CH₂ - C(O) - with the proviso, however, that at least one radical R², R³ and R⁴ does not represent hydrogen,
wherein said mixture comprises monoglycerides of 3-hydroxybutyric acid (3-BHB-MG), diglycerides of 3-hydroxybutyric acid (3-BHB-DG) and triglycerides of 3-hydroxybutyric acid (3-BHB-TG) in a weight ratio of 3-BHB-MG/3-BHB-DG/3-BHB-TG in the range of 10-80 / 10-70 / 0.1-20 and
wherein the reaction simultaneously produces the compound according to the general formula (IV)
R¹ - OH (IV)
wherein in the general formula (IV) the radical R¹ represents hydrogen or a C₁-C₄-alkyl, in particular a C₁-C4-alkyl, preferably methyl or ethyl, particularly preferably ethyl, wherein the compound according to the general formula (IV) is continuously withdrawn from the reaction.

2. The process according to claim 1,
wherein compound of the general formula (I) is used in racemic form or in the form of the (R)-enantiomer; and/or
wherein in the general formula (I) the radical R¹ represents ethyl; and/or
wherein 3-hydroxybutyric acid ethyl ester (ethyl 3-hydroxybutyrate) of the formula CH₃ - CH(OH) - CH₂ - C(O)OC₂H₅ is used as compound of the general formula (I).

3. The process according to claim 1 or claim 2,
wherein the catalyst is recycled after reaction; and/or
wherein the enzyme is selected from synthetases (ligases), catalases, esterases, lipases and combinations thereof; and/or
wherein the enzyme is derived from *Candida antarctica, Mucor miehei (Rhizomucor miehei), Thermomyces lanuginosus, Candida rugosa, Aspergillus oryzae, Pseudomonas cepacia, Pseudomonas fluorescens, Rhizopus delemar* and *Pseudomonas sp.* and combinations thereof, preferably *Candida antarctica, Mucor miehei (Rhizomucor miehei)* and *Thermomyces lanuginosus*; and/or
wherein the enzyme is used in immobilized form, in particular immobilized on a carrier, preferably on a polymeric carrier, preferably on a polymeric organic carrier, particularly preferably with hydrophobic properties, very particularly preferably on a poly(meth)acrylic resin-based carrier; and/or
wherein the enzyme is recycled after the reaction; and/or
wherein the reaction is carried out in the presence of an enzyme as catalyst at temperatures in the range of from 10 °C to 80 °C, in particular in the range of from 20 °C to 80 °C, preferably in the range of from 25 °C to 75 °C, more preferably in the range of from 45 °C to 75 °C, most preferably in the range from 50 °C to 70 °C; and/or
wherein the enzyme is employed in amounts, based on the total amount of the starting compounds of formulae (I) and (II), in the range of from 0.001 wt.% to 20 wt.%, in particular in the range of from 0.01 wt.% to 15 wt.%, preferably in the range of from 0.1 wt.% to 15 wt.%, more preferably in the range from 0.5 wt.% to 10 wt.%; and/or
wherein the reaction is carried out in the presence of an enzyme as catalyst at a pressure in the range of from 0.0001 bar to 10 bar, in particular in the range of from 0.001 bar to 5 bar, preferably in the range of from 0.01 bar to 2 bar, more preferably in the range of from 0.05 bar to 1 bar, very particularly at about 1 bar.

4. The process according to any one of the preceding claims,
wherein the compound of general formula (I) is used in molar amounts, relative to the hydroxyl groups of the glycerol of formula (II), in a range of from equimolar amount to a molar excess of 200 mol%, in particular in a range of from equimolar amount to a molar excess of 150 mol%, preferably in a range of from equimolar amount to a molar excess of 100 mol%; and/or
wherein the compound of the general formula (I) and the glycerol of the formula (II) are employed in a molar ratio of compound of the general formula (I)/glycerol of the formula (II) in a range of from 1:1 to 10:1, in particular in a range of from 2:1 to 8:1, preferably in a range of from 3:1 to 6:1.

5. The process according to any one of the preceding claims,
wherein the reaction product obtained is fractionated after the reaction, in particular fractionated by distillation;
in particular wherein the reaction product is at least separated into a first, in particular low-boiling fraction, which comprises a high proportion of mono- and diglycerides of 3-hydroxybutyric acid and a low proportion of triglycerides of 3-hydroxybutyric acid, and a second, in particular high-boiling fraction, which comprises a low proportion of monoglycerides of 3-hydroxybutyric acid and a high proportion of di- and triglycerides of 3-hydroxybutyric acid;
in particular wherein the first, in particular low-boiling fraction comprises a weight ratio of 3-BHB-MG/3-BHB-DG/3-BHB-TG in the range of 70 95 / 5-30 / 0.01-2, in particular in the range of 75-85 / 10 25 / 0-1, and/or in particular wherein the second, in particular high-boiling fraction comprises a weight-related ratio of 3-BHB-MG/3-BHB-DG/3-BHB-TG in the range of 5,30 / 20-80 / 5-40, in particular in the range of 5-20 / 40-75 / 10-30.

6. A reaction product obtainable according to the process of any one of claims 1 to 5.

7. A mixture of mono-, di- and triglycerides of 3-hydroxybutyric acid of the general formula (III)
CH₂(OR²) - CH(OR³) - CH₂(OR⁴) (III)
in which general formula (III) the radicals R², R³ and R⁴, in each case independently of one another, represent hydrogen or a radical of the formula CH₃ - CH(OH) - CH₂ - C(O) - with the proviso, however, that at least one radical R², R³ and R⁴ does not represent hydrogen,
said mixture comprising monoglycerides of 3 hydroxybutyric acid (3-BHB-MG), diglycerides of 3 hydroxybutyric acid (3-BHB-DG) and triglycerides of 3 hydroxybutyric acid (3-BHB-TG) in a weight ratio of 3-BHB-MG/3-BHB-DG/3-BHB-TG in the range of 10-80 / 10-70 / 0.1-20.

8. The mixture according to claim 7,
wherein the mixture comprises monoglycerides of 3-hydroxybutyric acid (3-BHB-MG), diglycerides of 3-hydroxybutyric acid (3 BHB-DG) and triglycerides of 3-hydroxybutyric acid (3-BHB-TG) in a weight ratio of 3-BHB-MG/3-BHB-DG/3-BHB-TG in the range of 20-70 / 20 60 / 0.5-15.

9. The mixture according to any one of claim 7 or claim 8,
wherein the mixture comprises a mixture of mono-, di- and triglycerides of 3-hydroxybutyric acid, wherein the mixture of mono-, di- and/or triglycerides of 3-hydroxybutyric acid comprises the mono-, di- and/or triglycerides of 3-hydroxybutyric acid in a weight ratio of 3-BHB-MG/3-BHB-DG/3-BHB-TG in the range of 70-95 / 5-30 / 0.01-2, in particular in the range of 75 85 / 10-25 / 0-1; or
wherein the mixture comprises a mixture of mono-, di- and triglycerides of 3-hydroxybutyric acid, wherein the mixture of mono-, di- and/or triglycerides of 3-hydroxybutyric acid comprises the mono-, di- and/or triglycerides of 3-hydroxybutyric acid in a weight ratio of 3-BHB-MG/3-BHB-DG/3-BHB-TG in the range of 5-30 / 20-80 / 5-40, in particular in the range of 5 20 / 40-75 / 10-30.

10. A pharmaceutical composition, in particular a drug or medicament, comprising a reaction product according to claim 6 and/or a mixture according to any one of claims 7 to 9.

11. The pharmaceutical composition according to claim 10 for use in the prophylactic and/or therapeutic treatment of diseases of the human or animal body, in particular diseases associated with a disorder of energy metabolism, in particular keto-body metabolism, such as in particular traumatic brain injury, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, lipometabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, gastrointestinal diseases such as inflammatory bowel diseases, especially ulcerative colitis and Crohn's disease, lyosomal storage diseases such as sphingolipidoses, especially Niemann-Pick disease, diabetes mellitus, and effects or side-effects of chemotherapy.

12. A reaction product according to claim 6 and/or mixture according to any one of claims 7 to 9 for use in the prophylactic and/or therapeutic treatment of diseases of the human or animal body, in particular diseases associated with a disorder of the energy metabolism, in particular keto-body metabolism, such as in particular craniocerebral trauma, stroke, hypoxia, cardiovascular diseases such as myocardial infarction, refeeding syndrome, anorexia, epilepsy, neurodegenerative diseases such as dementia, Alzheimer's disease, Parkinson's disease, multiple sclerosis and amyotrophic lateral sclerosis, lipometabolic diseases such as glucose transporter defect (GLUT1 defect), VL-FAOD and mitochondriopathies such as mitochondrial thiolase defect, Huntington's disease, cancers such as T-cell lymphomas, astrocytomas and glioblastomas, HIV, rheumatic diseases such as rheumatoid arthritis and arthritis urica, gastrointestinal diseases such as inflammatory bowel diseases, especially ulcerative colitis and Crohn's disease, lyosomal storage diseases such as sphingolipidoses, especially Niemann-Pick disease, diabetes mellitus, and effects or side-effects of chemotherapy.

13. A reaction product according to claim 6 and/or a mixture according to any one of claims 7 to 9 for use in catabolic metabolic conditions, in particular starvation, diets or low-carbohydrate diets.

14. A reaction product according to claim 6 and/or a mixture according to any one of claims 7 to 9 for use in the prophylactic and/or therapeutic treatment of catabolic metabolic disorders.

15. A food and/or food product comprising a reaction product according to claim 6 and/or a mixture according to any one of claims 7 to 9.

16. Use of a reaction product according to claim 6 and/or a mixture according to any one of claims 7 to 9 in a food and/or food product.

## Revendications

1. Procédé de préparation de glycérides de l'acide 3-hydroxybutyrique (acide béta-hydroxybutyrique, BHB ou 3-BHB),
où au moins un composé de la formule générale (I)
CH₃ - CH(OH) - CH₂ - C(O)OR¹ (I)
où, dans la formule générale (I), le radical R¹ représente l'hydrogène ou un alkyle en C₁-C₄, en particulier un alkyle en C₁-C₄, de préférence le méthyle ou l'éthyle, de manière particulièrement préférée l'éthyle,
est mis à réagir avec un glycérol (1,2,3-propanetriol) de formule (II)
CH₂(OH) - CH(OH) - CH₂(OH) (II)
la réaction étant effectuée en présence d'une enzyme comme catalyseur, et
la réaction étant effectuée en l'absence de solvants et/ou sans aucun solvant,
de manière à obtenir comme produit de réaction un mélange de mono-, di- et triglycérides de l'acide 3-hydroxybutyrique de la formule générale (III)
CH₂(OR²) - CH(OR³) - CH₂(OR⁴) (III)
où, dans la formule générale (III), les radicaux R², R³ et R⁴ représentent, indépendamment les uns des autres, l'hydrogène ou un radical de formule CH₃ - CH(OH) - CH₂ - C(O) -, à condition toutefois qu'au moins un radical R², R³ et R⁴ ne représente pas l'hydrogène,
où le mélange comprend des monoglycérides de l'acide 3-hydroxybutyrique (3-BHB-MG), des diglycérides de l'acide 3-hydroxybutyrique (3-BHB-DG) et des triglycérides de l'acide 3-hydroxybutyrique (3-BHB-TG) dans un rapport en poids de 3-BHB-MG/3-BHB-DG/3-BHB-TG dans la plage de 10-80 / 10-70 / 0,1-20 et
où, lors de la réaction, on obtient simultanément un composé selon la formule générale (IV)
R¹ - OH (IV)
où, dans la formule générale (IV), le radical R¹ représente l'hydrogène ou un alkyle en C₁ à C₄, en particulier un alkyle en C₁ à C₄, de préférence le méthyle ou l'éthyle, de manière particulièrement préférée l'éthyle, où le composé selon la formule générale (IV) est retiré en continu de la réaction.

2. Procédé selon la revendication 1,
où le composé de formule générale (I) est utilisé sous forme racémique ou sous forme d'énantiomère (R); et/ou
où, dans la formule générale (I), le reste R¹ représente l'éthyle; et/ou
où l'on utilise comme composé de formule générale (I) l'ester éthylique de l'acide 3-hydroxybutyrique (3-hydroxybutyrate d'éthyle) de la formule CH₃ - CH(OH) - CH₂ - C(O)OC₂H₅.

3. Procédé selon la revendication 1 ou la revendication 2,
où le catalyseur est recyclé après la réaction; et/ou
où l'enzyme est choisie parmi les synthétases (ligases), les catalases, les estérases, les lipases et leurs combinaisons; et/ou
où l'enzyme est dérivée de *Candida antarctica, Mucor miehei (Rhizomucor miehei), Thermomyces lanuginosus, Candida rugosa, Aspergillus oryzae, Pseudomonas cepacia, Pseudo*¬*monas fluorescens, Rhizopus delemar* et *Pseudomonas sp.* ainsi que leurs combinaisons, de préférence de *Candida antarctica, Mucor miehei (Rhizomucor miehei)* et *Thermomyces lanuginosus;* et/ou
où l'enzyme est utilisée sous forme immobilisée, en particulier immobilisée sur un support, de préférence sur un support polymère, de préférence sur un support organique polymère, de préférence avec des propriétés hydrophobes, tout particulièrement sur un support à base de résine poly(méth)acrylique; et/ou
où l'enzyme est recyclée après la réaction; et/ou
où la réaction est effectuée en présence d'une enzyme comme catalyseur à des températures de l'ordre de 10 °C à 80 °C, en particulier de l'ordre de 20 °C à 80 °C, de préférence de l'ordre de 25 °C à 75 °C, de manière particulièrement préférée de l'ordre de 45 °C à 75 °C, de manière tout particulièrement préférée de l'ordre de 50 °C à 70 °C; et/ou
où l'enzyme est utilisée en quantités, par rapport à la quantité totale des composés de départ de formules (I) et (II), dans la plage de 0,001 % en poids à 20 % en poids, en particulier dans la plage de 0,01 % en poids à 15 % en poids, de préférence dans la plage de 0,1 % en poids à 15 % en poids, de préférence dans la plage de 0,5 % en poids à 10 % en poids; et/ou
où la réaction est effectuée en présence d'une enzyme comme catalyseur à une pression dans la plage de 0,0001 bar à 10 bars, en particulier dans la plage de 0,001 bar à 5 bars, de préférence dans la plage de 0,01 bar à 2 bars, de manière particulièrement préférée dans la plage de 0,05 bar à 1 bar, tout particulièrement à environ 1 bar.

4. Procédé selon l'une quelconque des revendications précédentes,
où le composé de la formule générale (I) est utilisé en quantités molaires, par rapport aux groupes hydroxyle du glycérol de la formule (II), dans une plage allant d'une quantité équimolaire à un excès molaire de 200 % en moles, en particulier dans une plage allant d'une quantité équimolaire à un excès molaire de 150 % en moles, de préférence dans une plage allant d'une quantité équimolaire à un excès molaire de 100 % en moles; et/ou
où le composé de la formule générale (I) et le glycérol de formule (II) sont utilisés dans un rapport molaire composé de formule générale (I)/glycérol de formule (II) dans une plage de 1:1 à 10:1, en particulier dans une plage de 2:1 à 8:1, de préférence dans une plage de 3:1 à 6:1.

5. Procédé selon l'une quelconque des revendications précédentes,
où le produit de réaction obtenu est fractionné, en particulier fractionné par distillation, une fois la réaction effectuée;
en particulier où le produit de réaction est au moins séparé en une première fraction, en particulier à bas point d'ébullition, qui comprend une proportion élevée de mono- et diglycérides de l'acide 3-hydroxybutyrique et une faible proportion de triglycérides de l'acide 3-hydroxybutyrique, et une deuxième fraction, en particulier à haut point d'ébullition, qui comprend une faible proportion de monoglycérides de l'acide 3-hydroxybutyrique et une proportion élevée de di- et triglycérides de l'acide 3-hydroxybutyrique;
en particulier où la première fraction, en particulier à bas point d'ébullition, présente un rapport pondéral 3-BHB-MG/3-BHB-DG/3-BHB-TG dans la plage de 70-95 / 5-30 / 0,01-2, en particulier dans la plage de 75-85 / 10-25 / 0-1, et/ou en particulier où la deuxième fraction, en particulier à point d'ébullition élevé, comprend un rapport pondéral de 3-BHB-MG/3-BHB-DG/3-BHB-TG dans la plage de 5-30 / 20-80 / 5-40, en particulier dans la plage de 5-20 / 40-75 / 10-30.

6. Produit de réaction lequel peut être obtenu par le procédé selon l'une quelconque des revendications 1 à 5.

7. Mélange de mono-, di- et triglycérides de l'acide 3-hydroxybutyrique de la formule générale (III)
CH₂(OR²) - CH(OR³) - CH₂(OR⁴) (III)
où, dans la formule générale (III), les radicaux R², R³ et R⁴ représentent, chacun indépendamment les uns des autres, un atome d'hydrogène ou un radical de formule CH₃ - CH(OH) - CH₂ - C(O) -, à condition toutefois qu'au moins un radical R², R³ et R⁴ ne représente pas un atome d'hydrogène,
ledit mélange comprenant des monoglycérides de l'acide 3-hydroxybutyrique (3-BHB-MG), des diglycérides de l'acide 3-hydroxybutyrique (3-BHB-DG) et des triglycérides de l'acide 3-hydroxybutyrique (3 BHB-TG) dans un rapport en poids de 3-BHB-MG/3-BHB-DG/3-BHB-TG dans la plage de 10-80 / 10-70 / 0,1-20.

8. Mélange selon la revendication 7,
où le mélange comprend des monoglycérides de l'acide 3-hydroxybutyrique (3-BHB-MG), des diglycérides de l'acide 3-hydroxybutyrique (3-BHB-DG) et des triglycérides de l'acide 3-hydroxybutyrique (3-BHB-TG) dans un rapport en poids de 3-BHB-MG/3-BHB-DG/ 3-BHB-TG dans la plage de 20-70 / 20-60 / 0,5-15.

9. Mélange selon l'une quelconque des revendications 7 ou 8,
où le mélange comprend un mélange de mono-, di- et triglycérides de l'acide 3-hydroxybutyrique, où le mélange de mono-, di- et/ou triglycérides de l'acide 3-hydroxybutyrique comprend les mono-, di- et/ou triglycérides de l'acide 3-hydroxybutyrique dans un rapport en poids de 3-BHB-MG/3-BHB-DG/3-BHB-TG dans la plage de 70-95 / 5-30 / 0,01-2, en particulier dans la plage de 75-85 / 10-25 / 0-1; ou
où le mélange comprend un mélange de mono-, di- et triglycérides de l'acide 3-hydroxybutyrique, où le mélange de mono-, di- et/ou triglycérides de l'acide 3-hydroxybutyrique comprend les mono-, di- et/ou triglycérides de l'acide 3-hydroxybutyrique dans un rapport en poids de 3-BHB-MG/3-BHB-DG/3-BHB-TG dans la plage de 5-30 / 20-80 / 5-40, en particulier dans la plage de 5-20 / 40-75 / 10-30.

10. Composition pharmaceutique, en particulier médicament ou médicament, comprenant un produit de réaction selon la revendication 6 et/ou un mélange selon l'une quelconque des revendications 7 à 9.

11. Composition pharmaceutique selon la revendication 10, destinée à être utilisée dans le traitement prophylactique et/ou thérapeutique de maladies du corps humain ou animal, en particulier de maladies liées à un trouble du métabolisme énergétique, en particulier du métabolisme des corps cétoniques, comme notamment les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

12. Produit de réaction selon la revendication 6 et/ou mélange selon l'une quelconque des revendications 7 à 9, destiné à être utilisé dans le traitement prophylactique et/ou thérapeutique de maladies du corps humain ou animal, en particulier de maladies liées à un trouble du métabolisme énergétique, en particulier le métabolisme des corps cétoniques, comme en particulier les traumatismes crâniens, les accidents vasculaires cérébraux, les hypoxies, les maladies cardiovasculaires comme l'infarctus du myocarde, le syndrome de réalimentation, les anorexies, l'épilepsie, les maladies neurodégénératives comme la démence, la maladie d'Alzheimer, la maladie de Parkinson, la sclérose en plaques et la sclérose latérale amyotrophique, les maladies du métabolisme lipidique telles que le déficit en transporteur de glucose (déficit GLUT1), la VL-FAOD et les mitochondriopathies telles que le déficit en thiolase mitochondriale, la chorée de Huntington, les cancers tels que les lymphomes à cellules T, les astrocytomes et les glioblastomes, le VIH, les maladies rhumatismales telles que la polyarthrite rhumatoïde et l'arthrite urique, les maladies du tractus gastro-intestinal telles que les maladies inflammatoires chroniques de l'intestin, notamment la colite ulcéreuse et la maladie de Crohn, les maladies du stockage lyosomique telles que les sphingolipidoses, notamment la maladie de Niemann-Pick, le diabète sucré et les effets ou effets secondaires des chimiothérapies.

13. Produit de réaction selon la revendication 6 et/ou mélange selon l'une quelconque des revendications 7 à 9, destiné à être utilisé dans des situations de métabolisme catabolique, en particulier de famine, de régime ou de régime pauvre en hydrates de carbone.

14. Produit de réaction selon la revendication 6 et/ou un mélange selon l'une quelconque des revendications 7 à 9, destiné à être utilisé dans le traitement prophylactique et/ou thérapeutique de troubles métaboliques cataboliques.

15. Produit alimentaire et/ou nutritionnel comprenant un produit de réaction selon la revendication 6 et/ou un mélange selon l'une quelconque des revendications 7 à 9.

16. Utilisation d'un produit de réaction selon la revendication 6 et/ou d'un mélange selon l'une quelconque des revendications 7 à 9 dans un produit alimentaire et/ou nutritionnel.
